(19) Europäisches Patentamt
European Patent Office
Office européen des brevets

(11) EP 3 352 804 B1

(12) FASCICULE DE BREVET EUROPEEN

(45) Date de publication et mention de la délivrance du brevet:
**29.04.2020 Bulletin 2020/18**

(21) Numéro de dépôt: **16785544.4**

(22) Date de dépôt: **22.09.2016**

(51) Int Cl.:
***A61L 15/26*** *(2006.01)* ***A61L 15/58*** *(2006.01)*

(86) Numéro de dépôt international:
**PCT/FR2016/000144**

(87) Numéro de publication internationale:
**WO 2017/051083 (30.03.2017 Gazette 2017/13)**

# (54) ARTICLE ADHÉSIF À LA PEAU

AUF DER HAUT HAFTENDER GEGENSTAND

ITEM THAT ADHERES TO THE SKIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.09.2015 FR 1501990**

(43) Date de publication de la demande:
**01.08.2018 Bulletin 2018/31**

(73) Titulaire: **ELKEM SILICONES France SAS**
**69003 Lyon (FR)**

(72) Inventeurs:
- **CROS, Gaelle**
  **69360 Ternay (FR)**
- **MOINE, Caroline**
  **42290 Sorbiers (FR)**
- **MARROT, Sébastien**
  **69008 Lyon (FR)**

(56) Documents cités:
**WO-A1-2011/092404**

- **"Soft Skin Adhesive Gel for Scar Care and Wound Management HC2 2022 A&B Technical Data Sheet n° SIL 15 037 3 -March 2015", , mars 2015 (2015-03), XP055268817, Extrait de l'Internet: URL:http://www.silbione.com/wp-content/uploads/2014/01/Silbione_HC2_2022_AB_SIL150373.pdf [extrait le 2016-04-26]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 352 804 B1

## Description

La présente invention concerne un article adhésif utile pour une utilisation contre la peau et plus particulièrement comme :

- pansement adhésif à la peau, ou partie d'un tel pansement, en particulier destinés au retrait atraumatique d'une peau saine et d'une plaie, ou
- d'un dispositif de maintien d'accessoires médicaux, ou partie d'un tel dispositif, utilisés au contact de la peau de type capteur, sonde, cathéter ou aiguille.

L'utilisation des gels silicones dans les dispositifs médicaux destinés à être en contact avec la peau est à présent très répandue. En effet, les propriétés intrinsèques des gels silicones font qu'ils adhèrent rapidement à une peau sèche, mais ne collent pas sur la surface d'une plaie humide, ne provoquant pas ainsi de dommage lors de leurs retraits. Les gels silicones présentent aussi l'avantage de pouvoir être assemblés à un grand nombre de supports tout en étant inertes pour l'organisme, évitant ainsi tout problème de toxicité lorsqu'ils sont utilisés chez l'être humain. Les gels silicones sont entre autres utilisés pour le traitement des plaies ou cicatrices car ils apportent au dispositif médical des propriétés facilitant la guérison du patient en maintenant un milieu humide autour de la plaie et permettent ainsi de maintenir l'hydratation des tissus lésés. Ces propriétés sont bien documentées et comprennent le fait que les gels silicones ne laissent pas de particules ou de fibres dans la plaie, sont souples sur la peau et sont confortables.

Ainsi, de nombreux dispositifs médicaux intègrent des gels silicones en tant qu'adhésif à la peau ou en tant que couche de contact d'une plaie à traiter.

L'art antérieur décrit différents types de dispositifs médicaux comprenant ces gels silicones.

Par exemple, le brevet EP-A-0633758 de la société Moelnlycke AB décrit un pansement comprenant une couche de gel silicone hydrophobe, une couche de matériau support et un corps absorbant placé sur le côté du support, dans lequel le matériau support et la couche de gel comprennent des perforations pénétrantes qui coïncident mutuellement au moins dans la région du corps absorbant.

Le brevet EP-A-0633757 de la société Moelnlycke AB décrit le procédé de fabrication d'un tel pansement et implique une étape préalable qui consiste à souffler de l'air froid sur la face de dessous du matériau de support perforé recouvert par un mélange liquide de silicone précurseur du gel silicone, de sorte que les perforations du matériau support ne soient plus obstruées. Le courant d'air froid garantit que le mélange liquide de silicone précurseur du gel silicone ne commerce pas à durcir avant d'avoir le temps de se répandre sur le matériau de support. Le courant d'air froid traversant le matériau de support empêche également que le mélange liquide de silicone précurseur du gel silicone ne se répande dans les perforations dudit matériau. Le gel silicone est ensuite formé par une réaction de réticulation sous l'action de la chaleur.

Le brevet EP-A-2001424 de la société Brightwake Ltd décrit un stratifié adhésif amovible comprenant une couche structurelle comportant sur au moins une partie d'un côté de celle-ci un gel silicone hydrophobe et comportant sur au moins une partie de l'autre côté de celle-ci un adhésif sensible à la pression. La présence de l'adhésif sensible à la pression facilite l'assemblage des pansements composites qui comprennent ce type de stratifié. La présence de l'adhésif sensible à la pression permet l'attachement de composants du pansement secondaires, par exemple des matières absorbantes, au stratifié, et également de couches barrières imperméables aux fluides pour empêcher qu'un fluide, tel que l'exsudat de la plaie, ne s'échappe du pansement.

Le brevet EP-A-0300620 de la société Dow Corning SA décrit un pansement chirurgical, particulièrement adapté au traitement de brûlures, comprenant un film formé à partir d'un gel silicone comprenant une surface faisant face à la plaie et stratifié sur l'autre surface avec un film d'élastomère silicone.

Comme exemples de pansements vendus sur le marché utilisant des gels silicones, on peut citer :

- les pansements vendus sous la marque Mepitel® sont des pansements en tricot de polyamide enduit de gel de silicone destiné à être en contact avec la peau ;
- les pansements vendus sous la marque Allevyn® Life, Allevyn® Life Sacrum, Mepilex® Border et Allevyn® Life Heel mettant en œuvre une compresse de mousse hydrocellulaire composite placée entre une interface adhésive micro-perforée à base de gel de silicone, en contact avec la plaie, et un film externe hautement perméable et étanche à l'eau ;
- les pansements vendus sous la marque Cica-Care® ;
- les pansements vendus sous la marque Urgotul®, et
- les pansements vendus sous la marque Cerederm

Les gels silicones sont aussi largement utilisés dans les dispositifs de maintien d'articles médicaux utilisés au contact de la peau de type capteur, sonde, cathéter ou aiguille. Des exemples sont illustrés dans les demandes de

brevet FR-A1-2971971 et FR-A1-3004990 de la société Zodiac Automotive Division. Comme exemple de produit commercial on peut citer le Mepitel® Film.

WO 2011092404 décrit article adhésif pour la peau pour un usage médical ou paramédical comprenant un substrat plastique,de préférence un film de matière plastique, revêtu sur au moins sur une des deux faces par un primaire d'accrochage et une couche déposée sur le primaire d'accrochage constituée par un gel silicone préparé par réticulation d'une composition silicone comprenant au moins un polyorganosiloxane ayant en moyenne deux groupes alcényles liés à du silicium par molécule et aucun atome de silicium n'étant lié à plus d'un seul groupe alcényle G un composé hydrogéné du silicium H ayant au moins un composé hydrogéné du silicium H ayant au moins 2 atomes d'hydrogène liés à du silicium par molécule et un catalyseur d' hydrosylilation J à base de platine.

Cependant, les gels silicones restent fragiles lorsqu'ils sont soumis à des contraintes de de cisaillement, par exemple suite à des frottements répétés d'un vêtement sur un pansement appliqué sur la peau d'un patient ou lorsqu'ils sont utilisés comme adhésifs dans des dispositifs de fixation de sacs de stomie. Ainsi les différentes sollicitations subies par le gel le détériorent et entraînent l'apparition de trace de gel en périphérie du dispositif médical. La mesure de la résistance au cisaillement statique (« shear ») permet de quantifier la résistance d'un gel silicone soumis à des contraintes en cisaillement. La résistance au cisaillement statique est ainsi définie comme le temps nécessaire pour une aire de contact standard d'un gel silicone adhésif pour se séparer d'une surface standard plane lorsqu'il est soumis à un poids standard, par glissement dans une direction parallèle à cette surface lors de son application. Cette mesure donne une indication sur la capacité du gel silicone adhésif à résister à des forces statiques dans le plan et est déterminée selon la méthodologie décrite dans le document « FINAT Test Method no. 8 » (FINAT Technical Handbook 6th édition, 2001). Dans le présent mémoire, le test est réalisé avec des éprouvettes (support enduit par un gel) ayant une dimension de 25mm x 45mm et sur une plaque de type acier-inox. Ce test permet donc d'évaluer aussi le pouvoir cohésif ou la cohésivité d'un gel silicone adhésif. Plus la cohésivité du gel silicone est faible et plus il se détériore lorsqu'il est soumis à des contraintes de cisaillement statique ce qui se traduit par l'apparition de trace de gel sur le pourtour du dispositif médical. L'industrie des dispositifs médicaux, notamment ceux utilisant des gels silicones adhésif à la peau, est toujours dans l'attente de gels silicones ayant une résistance au cisaillement statique améliorée.

Cependant, l'amélioration de la cohésivité des gels silicones ne doit pas se faire au détriment de son pouvoir d'adhésion à la peau. En effet, il est important que les gels silicones adhèrent bien à la peau car ils servent également de moyen pour fixer l'article à la peau de l'utilisateur et pour le maintenir en place dans de nombreux dispositifs médicaux.

La force d'adhérence à la peau d'un gel silicone est évaluée par les deux propriétés suivantes :

- le «tack» ou collant instantané qui évalue l'habilité d'un gel silicone à adhérer rapidement à la peau, et
- le pouvoir adhésif ou pelage à un angle de 180° qui évalue la force nécessaire pour décoller un gel silicone d'une surface d'un matériau simulant le comportement d'une peau (feuille de papier bristol).

Pour apprécier et évaluer le collant instantané ou « tack », une méthode dite de «Probe Tack» est connue et est décrite dans la norme ASTM D2979. Ce test permet de mesurer l'adhésion instantanée de l'adhésif. Le principe est le suivant pour les gels silicones décrits dans le présent mémoire: un poinçon cylindrique à face plane est amené en contact avec le film d'adhésif qui est déposé sur le substrat. Le poinçon est maintenu ensuite en contact avec l'adhésif pendant un temps de contact de 1 seconde à une pression constante de 100 gf/$cm^2$. Ensuite, le poinçon est décollé à vitesse constante de 10 mm/s du film, et la force nécessaire pour séparer l'adhésif de la tige est mesurée et s'exprime en gf/$cm^2$ (l'énergie de décollement elle s'exprime en mJ/$cm^2$). Un gel silicone ayant un tack supérieur à 600 gf/$cm^2$ mesuré selon les conditions décrites ci-dessus est un gel silicone particulièrement recherché et adapté pour une utilisation dans les dispositifs médicaux au contact de la peau.

Le pouvoir adhésif ou pelage (« peel » en anglais) d'un gel silicone à la peau est la force nécessaire pour le décoller d'une feuille de papier bristol, simulant la peau, de dimension bien définie, à un angle de 180° et à une vitesse constante de 300 mm/min et avec l'aide d'une cellule de force de 10N (newtons) dans le cas des gels silicones. Il est évalué par la méthodologie décrite le document FINAT Test Method n°1 (FINAT Technical Handbook 6th édition, 2001). Ainsi, un article adhésif à la peau ayant des dimensions définies (dans le présent mémoire de 40 mm x150 mm) et comprenant un support sur lequel est enduite une couche de gel silicone est appliqué en mettant en contact le gel silicone sur une feuille de papier bristol. L'article est ensuite décollé et la force est mesurée et ramenée à la largeur de l'article et s'exprime en N/cm.

Ainsi, la présente invention a pour objet de proposer de nouveaux articles adhésifs à la peau comprenant un support sur lequel est enduit un gel silicone adhésif présentant une résistance au cisaillement statique améliorée de manière à permettre une utilisation prolongée dans des dispositifs médicaux soumis à ce type de contraintes, par exemple suite à des frottements répétés d'un vêtement sur un pansement appliqué sur la peau d'un patient, ou à des tensions élevées, comme par exemple lorsqu'ils sont utilisés comme adhésifs dans des dispositifs de fixation de sacs de stomie.

Un autre objectif de la présente invention est de proposer de nouveaux articles adhésifs à la peau comprenant

un gel silicone adhésif présentant de bonnes propriétés d'adhésion.

Ces objectifs sont atteints par l'invention qui concerne un article adhésif à la peau comprenant :

- un support **S** ayant une face de dessus **S1** et une face de dessous **S2,**
- éventuellement au moins un primaire d'accrochage **C1** appliqué sur au moins une partie ou sur la totalité de la face de dessus **S1** dudit support S,
- au moins une couche **D1** appliquée de manière continue ou discontinue sur la face de dessus **S1** dudit support **S** ou sur ledit primaire d'accrochage **C1** lorsqu'il est présent, et qui est constituée par un gel silicone **E** adhésif à la peau ayant les propriétés suivantes:

  a) une pénétrabilité comprise entre 80 mm/10 et 300 mm/10, de préférence comprise entre 80 mm/10 et 200 mm/10, mesurée selon la norme NF ISO 2137 avec un pénétromètre ayant une tige et un cône et dont la somme des poids est égale à 62,5 g, et

  b) un collant instantané ou «tack » compris entre 600 gf/cm$^2$ et 900 gf/cm$^2$, de préférence entre 700 gf/cm$^2$ et 850 gf/cm$^2$ pour une couche de 200 g/m$^2$ enduite sur un support en PET ayant une épaisseur de 36 $\mu$m et mesuré selon la norme ASTM D2979,

  c) un pouvoir adhésif compris entre 1,05 N/cm et 1,25 N/cm, de préférence compris entre 1,10 N/cm et 1,20 N/cm, pour une couche de 200 g/m$^2$ enduite sur un support en PET ayant une épaisseur de 36 $\mu$m et mesuré selon la méthode de test FINAT no. 1 par mise en contact sur une bande de papier bristol avec un angle de pelage de 180°, et

  d) une résistance au cisaillement statique à 23°C ou « shear » supérieure à 3 heures mesurée selon la méthode de test FINAT no. 8 pour une couche de 200 g/m$^2$ enduite sur un support en PET ayant une épaisseur de 36 $\mu$m ; et

- éventuellement au moins une couche de protection **F** constituée d'un matériau de protection pelable et appliquée sur ladite couche **D1,**

le dit gel silicone **E** étant obtenu par réticulation d'une composition silicone **X** comprenant :

1) au moins un organopolysiloxane **A** comportant, par molécule, au moins deux radicaux alcényles en $C_2$ à $C_6$ liés chacun à un atome de silicium, et constitué :

(i) d'au moins deux motifs de formule (A1) :

$$(Y)a(Z)_bSiO_{(4\ -(a+b)/2} \qquad (A1)$$

dans laquelle :

- Y représente un groupe alcényle en $C_2$ à $C_6$, et de préférence un groupe vinyle,
- Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle;
- a et b représentent des nombres entiers, a valant 1, 2 ou 3, b valant 0, 1 ou 2 et (a+b) valant 2 ou 3;

(ii) et éventuellement d'au moins un motif de formule (A2) :

$$(Z)_cSiO_{(4-c)/2} \qquad (A2)$$

dans laquelle :

- Z a la même signification que ci-dessus, et
- c représente un nombre entier valant 2 ou 3.

2) au moins un organopolysiloxane **B** comportant, par molécule, au moins deux atomes d'hydrogène liés chacun à un atome de silicium, et de préférence au moins trois atomes d'hydrogène liés chacun à un atome de silicium,

3) au moins un catalyseur **C** d'hydrosilylation,

4) au moins un inhibiteur **D** de la réaction d'hydrosilylation,

5) éventuellement au moins un additif **K,** et

6) entre 1,5 % et 3,5 % en poids, et de préférence entre 1,75 % et 3,0 % en poids, par rapport au poids total de la composition silicone **X** d'au moins une résine silicone **Z** ayant des groupements alcényles liés à des atomes de silicium et comprenant :

a) au moins un motif siloxyle de formule **(I)** :

$$Y\,R_aSiO_{\frac{(3-a)}{2}}$$

**(I)**

dans laquelle :

- Y représente un groupe alcényle en $C_2$ à $C_6$, et de préférence un groupe vinyle,
- R est un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle, et
- a = 0, 1 ou 2, et de préférence a = 1 ou 2

b) au moins un motif siloxy de formule **(II)** :

$$R_bSiO_{\frac{(4-b)}{2}}$$

**(II)**

dans laquelle R ayant la même définition que ci-dessus et b = 1, 2 ou 3 ; et

c) au moins un motif siloxy Q de formule **(III)** :

$$SiO_{\frac{4}{2}}$$

**(III)**

avec les conditions suivantes :

a) les quantités en poids des organopolysiloxanes **A, B** et **Z** sont déterminées de manière à ce que la valeur du rapport $RH_{alk} = n_H/t_{Alk}$ soit comprise dans l'intervalle suivant : $0{,}10 \leq RH_{alk} \leq 0{,}80$, de préférence dans l'intervalle suivant $0{,}20 \leq RH_{alk} \leq 0{,}80$, et encore plus préférentiellement dans l'intervalle suivant $0{,}20 \leq RH_{alk} \leq 0{,}75$, avec $n_H$ = nombre de mole d'atome d'hydrogène directement lié à un atome de silicium de l'organopolysiloxane **B** et $t_{Alk}$ = nombre de mole d'alcényle directement lié à un atome de silicium de l'organopolysiloxane **A** et de la résine silicone **Z**, et

b) les viscosités et les quantités en poids des constituants de la composition silicone **X** sont choisies de manière à ce que la viscosité de la composition silicone **X** soit comprise entre 200 mPa.s et 100000 mPa.s à 25°C, et de préférence comprise entre 200 mPa.s et 80000 mPa.s à 25°C.

La Demanderesse a mis en œuvre d'importants moyens de recherche et de nombreuses expérimentations pour atteindre cet objectif parmi d'autres. Et au terme de cela, elle a eu le mérite de trouver, de manière tout à fait surprenante et inattendue, qu'en ajoutant dans une composition silicone, précurseur d'un gel silicone par une réaction de polyaddition, une quantité en poids choisie dans un intervalle spécifique d'une résine silicone ayant des motifs siloxy Q de formule (III et des groupements alcényles liés à des atomes de silicium permet, non seulement d'améliorer très nettement sa résistance au cisaillement statique du gel silicone obtenu, mais aussi d'améliorer son aptitude à adhérer à la peau.

L'amélioration de la résistance au cisaillement (« shear »), pouvant atteindre un facteur 10, et de l'aptitude à adhérer à la peau des gels silicones selon l'invention, permet une utilisation efficace et prolongée dans des dispositifs médicaux soumis à des contraintes en cisaillement statique, par exemple suite à des frottements répétés d'un vêtement

sur un pansement appliqué sur la peau d'un patient, ou lorsqu'ils sont utilisés comme adhésifs dans des dispositifs de fixation de sacs de stomie. Il présente donc l'avantage de ne pas laisser de résidus sur la peau même lorsqu'il est utilisé dans des dispositifs médicaux subissant des contraintes en cisaillement statique importantes.

Au sens de la présente invention, l'expression « gel silicone » désigne un produit silicone réticulé caractérisé notamment par un taux de pénétration (ou « pénétrabilité ») compris entre 50 et 500 dixièmes de mm. Elle se mesure par pénétrométrie selon la norme NF ISO 2137, à l'aide d'un pénétromètre modèle PNR 12 Petrotest avec un poids total de la tige et du cône fixé à 62,5 g. La pénétrabilité au cône d'un gel silicone est déterminée à 25 °C en mesurant la profondeur de pénétration du cône dans l'échantillon, celle-ci étant obtenue en libérant l'ensemble cône du pénétromètre et en laissant le cône agir pendant 5 secondes.

Les gels silicones selon l'invention présentent eux un taux de pénétration compris entre 80 mm/10 et 300 mm/10, et de préférence compris entre 80 mm/10 et 200 mm/10.

Toutes les viscosités dont il est question dans le présent exposé correspondent à une grandeur de viscosité dynamique à 25°C dite "Newtonienne", c'est-à-dire la viscosité dynamique qui est mesurée, de manière connue en soi, avec un viscosimètre Brookfield à un gradient de vitesse de cisaillement suffisamment faible pour que la viscosité mesurée soit indépendante du gradient de vitesse.

Selon un mode de réalisation préféré, l'invention concerne un article adhésif à la peau comprenant :

- un support **S** ayant une face de dessus **S1** et une face de dessous **S2,**
- au moins un primaire d'accrochage **C1** appliqué sur au moins une partie ou sur la totalité de la face de dessus **S1** dudit support S,
- au moins une couche **D1** appliquée de manière continue ou discontinue sur ledit primaire d'accrochage **C1**, et qui est constituée par un gel silicone **E** adhésif à la peau ayant les propriétés suivantes :

  a) une pénétrabilité comprise entre 80 mm/10 et 300 mm/10, de préférence comprise entre 80 mm/10 et 200 mm/10, mesurée selon la norme NF ISO 2137 avec un pénétromètre ayant une tige et un cône et dont la somme des poids est égale à 62,5 g, et
  b) un collant instantané ou «tack » compris entre 600 gf/$cm^2$ et 900 gf/$cm^2$, de préférence entre 700 gf/$cm^2$ et 850 gf/$cm^2$ pour une couche de 200 g/$m^2$ enduite sur un support en PET ayant une épaisseur de 36 μm et mesuré selon la norme ASTM D2979,
  c) un pouvoir adhésif compris entre 1,05 N/cm et 1,25 N/cm, de préférence compris entre 1,10N/cm et 1,20 N/cm, pour une couche de 200 g/$m^2$ enduite sur un support en PET ayant une épaisseur de 36 μm et mesuré selon la méthode de test FINAT no. 1 par mise en contact sur une bande de papier bristol avec un angle de pelage de 180°, et
  d) une résistance au cisaillement statique à 23°C ou « shear » supérieure à 3 heures mesurée selon la méthode de test FINAT no. 8 pour une couche de 200 g/$m^2$ enduite sur un support en PET ayant une épaisseur de 36 μm ; et

- éventuellement au moins une couche de protection **F** constituée d'un matériau de protection pelable et appliquée sur ladite couche **D1,**

le dit gel silicone **E** étant obtenu par réticulation d'une composition silicone **X** comprenant :

1) au moins un organopolysiloxane **A** comportant, par molécule, au moins deux radicaux alcényles en $C_2$ à $C_6$ liés chacun à un atome de silicium, et constitué :

(i) d'au moins deux motifs siloxy de formule (A1) :

$$(Y)a(Z)_bSiO_{(4-(a+b)/2} \qquad (A1)$$

dans laquelle :

- Y représente un groupe alcényle en $C_2$ à $C_6$, et de préférence un groupe vinyle,
- Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle;
- a et b représentent des nombres entiers, a valant 1, 2 ou 3, b valant 0, 1 ou 2 et (a+b) valant 2 ou 3;

(ii) et éventuellement d'au moins un motif siloxy de formule (A2) :

$$(Z)_cSiO_{(4-c)/2} \quad (A2)$$

dans laquelle :

- Z a la même signification que ci-dessus, et
- c représente un nombre entier valant 2 ou 3.

2) au moins un organopolysiloxane **B** comportant, par molécule, au moins deux atomes d'hydrogène liés chacun à un atome de silicium, et de préférence au moins trois atomes d'hydrogène liés chacun à un atome de silicium,
3) au moins un catalyseur **C** d'hydrosilylation,
4) au moins un inhibiteur **D** de la réaction d'hydrosilylation,
5) éventuellement au moins un additif **K,** et
6) entre 1,5 % et 3,5 % en poids, et de préférence entre 1,75 % et 3,0 % en poids, par rapport au poids total de la composition silicone **X** d'au moins une résine silicone **Z** ayant des groupements alcényles liés à des atomes de silicium et comprenant :

a) au moins un motif siloxy de formule **(I)** :

$$Y\,R_aSiO_{\frac{(3-a)}{2}}$$

**(I)**

dans laquelle :

- Y représente un groupe alcényle en $C_2$ à $C_6$, et de préférence un groupe vinyle,
- R est un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle, et
- a = 0, 1 ou 2, et de préférence a = 1 ou 2

b) au moins un motif siloxy de formule **(II)** :

$$R_bSiO_{\frac{(4-b)}{2}}$$

**(II)**

dans laquelle R ayant la même définition que ci-dessus et b = 1, 2 ou 3 ; et
c) au moins un motif siloxy Q de formule (III) :

$$SiO_{\frac{4}{2}}$$

**(III)**

avec les conditions suivantes :

a) les quantités en poids des organopolysiloxanes **A, B** et **Z** sont déterminées de manière à ce que la valeur du rapport $RH_{alk} = n_H/t_{Alk}$ soit comprise dans l'intervalle suivant : $0,10 \leq RH_{alk} \leq 0,80$, de préférence dans l'intervalle suivant $0,20 \leq RH_{alk} \leq 0,80$, et encore plus préférentiellement dans l'intervalle suivant $0,20 \leq RH_{alk} \leq 0,75$, avec $n_H$ = nombre de mole d'atome d'hydrogène directement lié à un atome de silicium de l'organopolysiloxane **B** et $t_{Alk}$ = nombre de mole d'alcényle directement lié à un atome de silicium de l'organopolysiloxane **A** et de la résine silicone **Z,** et

b) les viscosités et les quantités en poids des constituants de la composition silicone **X** sont choisies de manière à ce que la viscosité dynamique à 25°C de la composition silicone **X** soit comprise entre 200 mPa.s et 100000 mPa.s, et de préférence comprise entre 200 mPa.s et 80000 mPa.s.

Selon un mode de réalisation préféré, le support **S** est un textile tissé, non-tissé ou tricoté, ou un film de matière plastique.

Par le terme « non-tissé », on entend toute structure constituée de matières textiles, comme des fibres, des filaments continus ou des fils coupés, quelle qu'en soit la nature ou l'origine, formée en voile par un moyen quelconque, et liée par tout moyen, excluant l'entrelacement de fils.

Les textiles non-tissés sont des produits ayant l'apparence de textiles, poreux, composés principalement de fibres et sont fabriqués par des procédés autres que la filature, le tissage, le tricotage ou le nouage.

Une grande variété de matières plastiques peut être appropriée pour une utilisation en tant que support **S** selon l'invention. Des exemples comprennent : le polychlorure de vinyle, le polypropylène, la cellulose régénérée, le polyéthylène téréphtalate (PET) et le polyuréthane, en particulier le polyuréthane fondu-soufflé.

De préférence, le support **S** est un film souple perforé en polyuréthane ou un film souple continu en polyuréthane. Ce film souple en polyuréthane peut-être fabriqué à partir de polyuréthane fondu soufflé.

Préférentiellement, on utilise un film souple en polyuréthane transparent ou translucide. Lorsque l'article adhésif a un usage de pansement, l'utilisation d'un film transparent ou translucide présente l'avantage de permettre l'observation de la plaie, de la blessure ou du site d'entrée d'un cathéter sur lequel le pansement doit être centré.

De préférence, le dit support **S** est un film souple en polyuréthane ayant une épaisseur de 5 à 600 $\mu$m, de préférence de 5 à 250 $\mu$m et encore plus préférentiellement de 10 à 100 $\mu$m.

Comme exemple de film souple en polyuréthane on peut citer ceux qui sont utilisés dans les pansements commercialisés par la société Smith & Nephew sous la marque Opsite®, ou par la société 3M sous la marque Tegaderm® ou encore par les Laboratoires URGO sous la marque Optiskin®. Ces pansements sont constitués d'un film mince (de l'ordre de 20 à 50 $\mu$m) transparent de polyuréthane adhésivé. Leur transparence permet un contrôle visuel de la zone à traiter.

Comme autre exemple de film souple en polyuréthane on peut aussi citer ceux commercialisés sous les marques Platilon® vendus par la société Bayer Material Science et Inspire® vendus par la société Coveris Advanced Coatings.

Selon un mode de réalisation préféré, le support **S** est un film souple continu qui est perméable à l'air et imperméable aux fluides.

Le film peut présenter un taux de transmission de vapeur d'eau (MVTR : « Moisture Vapor Transmission Rate») variable suivant l'application visée. Une technique de mesure du taux de transmission de vapeur d'eau en contact liquide est décrite dans la norme NF-EN 13726-2. De préférence, le film souple en polyuréthane sera choisi de manière à obtenir un pansement ayant un taux de transmission de vapeur d'eau supérieur à 300 g/m$^2$/24 heures, de préférence supérieur ou égal à 600 g/m$^2$/24 heures, de préférence encore supérieur ou égal à 1000 g/m$^2$/24 heures.

Selon un autre mode de réalisation particulier, l'invention concerne un article adhésif à la peau caractérisé en ce que le support **S** est un film souple en polyuréthane et comporte sur au moins une partie de la face de dessous **S2** un adhésif sensible à la pression.

Selon une variante avantageuse de l'invention le film souple continu en polyuréthane est perforé de manière à pouvoir favoriser la circulation des exsudats.

Ainsi l'article adhésif selon l'invention est selon un mode de réalisation particulier un stratifié adhésif amovible et présente l'avantage de pouvoir être utilisé en tant que couche de contact avec la peau dans différents types de dispositifs médicaux tels que des pansements par exemple.

L'adhésif sensible à la pression peut être n'importe lequel des nombreux adhésifs sensibles à la pression connus de l'art. Ces adhésifs, généralement sous une forme anhydre et sans solvant, sont collants de façon permanente à température ambiante et adhèrent fermement à une variété de surfaces dissemblables lors d'un contact simple, sans qu'il y ait besoin d'utiliser plus que la pression d'un doigt ou de la main. Ils ne nécessitent pas d'activation par de l'eau, un solvant ou de la chaleur afin d'avoir une forte force adhésive de maintien. Des exemples d'adhésifs sensibles à la pression comprennent les adhésifs de caoutchouc/résine, qui sont des mélanges de matière caoutchouteuse et de résine dure, et les adhésifs acryliques (ou d'acrylate). La classe actuellement préférée d'adhésifs sensibles à la pression pour une utilisation dans la présente invention est celle des adhésifs acryliques.

Selon un mode de réalisation particulier, le support **S** est un film souple perforé en polyuréthane ou un film souple continu en polyuréthane ayant une face de dessus **S1** et une face de dessous **S2** et qui est imperméable à l'air et aux fluides dans ses parties comprises entre les perforations.

Dans ce mode de réalisation particulier, il est avantageux que les perforations du support **S** soient circulaires et aient un diamètre de 50 $\mu$m à 10 mm.

Comme exemple de primaire d'accrochage **C1** ou primaire d'adhérence, on peut citer :

- Les primaires formulés en milieu solvant. Un exemple est décrit dans la demande de brevet WO 2011/092404 de la société Bluestar Silicones France où un primaire est constitué d'une matière active (huile organopolysiloxane à fonction hydrogénosilylé (SiH) et Si-alcényle ou une résine silicone ayant des fonctions hydrogénosilylé) diluée dans un solvant silicone (cyclopentasiloxane),
- Les primaires décrits dans la demande de brevet français déposée sous le n° FR 15 01350 au nom de la société Bluestar Silicones, et
- les primaires en élastomère de silicone qui sont préparés à partir de compositions précurseurs réticulant par une réaction d'hydrosilylation comprenant des promoteurs d'adhésion qui sont le plus souvent des silanes qui permettent d'améliorer l'adhésion sur divers substrats (en polyamide, en polyester ou en polyuréthane).

La couche de protection **F** constituée d'un matériau de protection pelable peut être constituée d'une ou plusieurs parties pelables avant usage. Cette couche de protection recouvre de préférence toute la surface du gel de l'article adhésif et pourra être de tout matériau couramment utilisé comme protection par l'homme du métier dans le domaine des pansements. Elle pourra se présenter par exemple sous forme de film par exemple un film de polyoléfine tels que le polyéthylène ou le polypropylène ou un film de polyester. Ce film peut-être avantageusement traité sur au moins l'une de ses faces par un composé siliconé comme un silane, un composé fluoré, ou un composé siliconé et fluoré. Le choix du matériau est en général adapté à la nature du gel de silicone. La couche de protection **F** constituée d'un matériau de protection pelable est de préférence dotée d'une épaisseur comprise entre 10 et 100 $\mu$m, par exemple de l'ordre de 50 $\mu$m.

Selon un autre mode de réalisation particulier, l'article adhésif à la peau selon l'invention est caractérisé en ce qu'il comprend une ou plusieurs couches **N** comprenant un corps absorbant O, éventuellement séparées par une ou plusieurs couches intermédiaires **P,** placée(s) sur le support **S** du côté de la face de dessous **S2** du support **S.**

De préférence, le corps absorbant **O** est choisi parmi le groupe constitué par: une mousse hydrophile, un tampon en tissu, un hydrogel, un hydrocolloïde et un alginate. De préférence, le corps absorbant **O** est une mousse en polyuréthane.

De préférence, les quantités de gel silicone **E** selon l'invention sont déterminées de manière à obtenir des enductions ayant une teneur en gel silicone comprise entre 20 et 500 g/$m^2$ de support, de préférence entre 40 et 250 g/$m^2$ et encore plus préférentiellement entre 80 et 350 g/$m^2$.

Comme technique pour déposer la composition **X** selon l'invention, on peut citer par exemple les techniques d'enduction réalisées par racle, en particulier par racle sur cylindre, racle en l'air et racle sur tapis, ou par foulardage, c'est-à-dire par exprimage entre deux rouleaux, ou encore par rouleau lécheur, cadre rotatif, rouleau inverse "reverse roll", transfert ou pulvérisation.

Comme autre technique d'enduction on peut citer la technique de couchage au rideau. Le couchage au rideau est un procédé d'application d'un liquide de couchage sur un article ou un support. Le couchage au rideau est caractérisé par la formation d'un rideau tombant librement d'un liquide de couchage qui tombe de la lèvre du Hopper et, sous l'effet de la gravité, vient rencontrer l'article se déplaçant à travers le rideau pour former un couchage (ou une enduction). Cette technique a été très utilisée dans le domaine de la préparation de supports argentiques photosensibles multicouches (voir par exemple les brevets US-3508947, US3508947 ou EP537086).

Il est connu que la qualité du couchage dépend de la qualité du rideau tombant librement. Il est préférable que le rideau présente un écoulement laminaire stable depuis l'endroit où il se forme jusqu'à la ligne rencontre avec le support en mouvement. A défaut, la tension superficielle amènera le rideau à se contracter vers l'intérieur et à interrompre l'écoulement laminaire. Afin d'éviter ce problème, il est connu d'employer des guides de bord pour saisir le rideau tombant librement au niveau de ses bords et empêcher sa contraction vers l'intérieur en raison de la tension superficielle. Des exemples de tels systèmes sont décrits dans les brevets US4933215, US4479987, US4974533, US3632374, US4479987, EP537086 et US4830887.

Selon l'invention, l'organopolysiloxane **A** comporte, par molécule, au moins deux radicaux alcényles en $C_2$ à $C_6$ liés chacun à un atome de silicium, et est constitué :

(i) d'au moins deux motifs de formule (A1) :

$$(Y)a(Z)_bSiO_{(4-(a+b)/2} \qquad (A1)$$

dans laquelle :

- Y représente un radical monovalent contenant de 2 à 6 atomes de carbone, ayant au moins un groupe alcényle,
- Z représente un radical monovalent contenant de 1 à 20 atomes de carbone et ne comprend pas de groupe alcényle;
- a et b représentent des nombres entiers, a valant 1, 2 ou 3, b valant 0, 1 ou 2 et (a+b) valant 2 ou 3 ;

(ii) et éventuellement d'au moins un motif de formule (A2) :

$$(Z)_cSiO_{(4-c)/2} \qquad (A2)$$

dans laquelle :

- Z a la même signification que ci-dessus, et
- c représente un nombre entier valant 2 ou 3.

Il est entendu dans la formule (A1) et dans la formule (A2) ci-dessus que, si plusieurs radicaux Y et Z sont présents, ils peuvent être identiques ou différents les uns des autres.

Dans la formule (A1), le symbole a peut préférentiellement valoir 1 ou 2, plus préférentiellement 1. De plus, dans la formule (A1) et dans la formule (A2), Z peut représenter préférentiellement un radical monovalent choisi dans le groupe constitué par un groupe alkyle ayant 1 à 8 atomes de carbone, éventuellement substitué par au moins un atome d'halogène, et un groupe aryle. Z peut avantageusement représenter un radical monovalent choisi dans le groupe constitué par méthyle, éthyle, propyle, 3,3,3-trifluoropropyle, xylyle, tolyle et phényle. En outre, dans la formule (A1), Y peut avantageusement représente un radical choisi dans le groupe constitué par vinyle, propényle, 3-butényle et 5-hexényle. De préférence, le symbole Y est un vinyle et le symbole Z est un méthyle.

Lorsqu'il s'agit d'organopolysiloxanes linéaires, l'organopolysiloxane A est essentiellement constitués :

- de motifs siloxyles « D » choisi parmi les motifs de formules $(Y)_2SiO_{2/2}$, $(Y)(Z)SiO_{2/2}$ et $(Z)_2SiO_{2/2}$ ;
- et de motifs siloxyles « M » choisis parmi les motifs de formules $(Y)_3SiO_{1/2}$, $(Y)_2(Z)SiO_{1/2}$, $(Y)(Z)_2SiO_{1/2}$ et $(Z)_3SiO_{2/2}$
- avec la condition qu'au moins deux motifs siloxy comprenant un groupe Y soit présent dans la structure chimique de l'organopolysiloxane **A,**
- et les symboles Y et Z sont tels définis ci-dessus.

A titre d'exemples de motifs « D », on peut citer les groupes diméthylsiloxy, méthylphénylsiloxy, méthylvinylsiloxy, méthylbuténylsiloxy, méthylhexénylsiloxy, méthyldécénylsiloxy et méthyldécadiénylsiloxy.

A titre d'exemple de motifs « M », on peut citer les groupes triméthylsiloxy, diméthylphénylsiloxy, diméthylvinylsiloxy et diméthylhexénylsiloxy.

Ces organopolysiloxanes, en particulier lorsqu'ils sont linéaires, peuvent être des huiles ayant une viscosité dynamique à 25°C comprise entre 50 mPa.s et 120 000 mPa.s, préférentiellement entre 100 mPa.s et 80 000 mPa.s.

Lorsqu'il s'agit d'un organopolysiloxane cyclique, celui-ci peut être constitué de motifs siloxyles « D » choisi parmi les motifs de formules $Y_2SiO_{22}$, $YZSiCO_{2/2}$ et $Z_2SiO_{2/2}$, avec la condition qu'au moins deux motifs siloxy comprenant un groupe Y soit présent dans la structure chimique de l'organopolysiloxane **A.** Des exemples de tels motifs « D » sont décrits ci-dessus. Ce polyorganosiloxane cyclique peut avoir une viscosité dynamique à 25°C comprise entre 1 mPa.s et 5 000 mPa.s.

Des exemples d'organopolysiloxane **A** sont :

- les polydiméthylsiloxanes à extrémités diméthylvinylsilyles ;
- les poly(méthylphénylsiloxane-co-diméthylsiloxane) à extrémités diméthylvinylsilyles ;
- les poly(vinylméthylsiloxane-co-diméthylsiloxane) à extrémités diméthylvinylsilyles ;
- les poly(diméthylsiloxane-co-vinylméthylsiloxane) à extrémités triméthylsilyles ; et
- les polyméthylvinylsiloxanes cycliques.

Les organopolysiloxane **A** qui sont des polydiméthylsiloxanes à extrémités diméthylvinylsilyles ayant une viscosité dynamique à 25°C comprise entre 50 mPa.s et 120 000 mPa.s, et de préférence comprise entre 100 mPa.s et 80000 mPa.s sont particulièrement avantageux. La formule de ce type d'organopolysiloxane **A** particulièrement avantageux est $M^{Vi}D_xM^{Vi}$, formule dans laquelle :

- $M^{Vi}$ = motif siloxy de formule : $(vinyle)(CH_3)_2SiO_{1/2}$
- D = motif siloxyl de formule : $(CH_3)_2SiO_{2/2}$, et

x est un nombre compris entre 0 et 1000, et de préférence compris entre 5 et 1000.

L'organopolysiloxane **B** selon l'invention est porteur d'au moins deux atomes d'hydrogène liés à des atomes de silicium, et de préférence d'au moins trois atomes d'hydrogène liés à des atomes de silicium. Selon un mode de réalisation préféré, cet organopolysiloxane **B** comprend :

(i) au moins deux motifs de formule (B1), et de préférence au moins trois motifs de formule (B1) :

$$(H)_d(L)_eSiO_{(4-(d+e))/2} \qquad (B1)$$

dans laquelle :

- L représente un radical monovalent différent d'un atome d'hydrogène,
- H représente l'atome d'hydrogène,
- d et e représentent des nombres entiers, d valant 1 ou 2, e valant 0, 1 ou 2 et (d+e) valant 1, 2 ou 3 ; et de préférence la somme (d+e) est égale à 2 ou 3.

et éventuellement d'autres motifs de formule (B2) :

$$(L)_fSiO_{(4-f)/2} \qquad (B2)$$

dans laquelle :

- L a la même signification que ci-dessus, et
- f représente un nombre entier valant 0, 1, 2 ou 3, et de préférence f est égal à 2 ou 3.

Il est entendu dans la formule (B1) et dans la formule (B2) ci-dessus que si plusieurs groupes L sont présents, ils peuvent être identiques ou différents les uns des autres. Dans la formule (B1), le symbole d peut préférentiellement valoir 1. De plus, dans la formule (B1) et dans la formule (B2), L peut représenter de préférence un radical monovalent choisi dans le groupe constitué par un groupe alkyle ayant 1 à 8 atomes de carbone, éventuellement substitué par au moins un atome d'halogène, et un groupe aryle. L peut avantageusement représenter un radical monovalent choisi dans le groupe constitué par méthyle, éthyle, propyle, 3,3,3-trifluoropropyle, xylyle, tolyle et phényle. Des exemples de motifs de formule (B1) sont les suivants : $H(CH_3)_2SiO_{1/2}$, $H(CH_3)SiO_{2/2}$ et $H(C_6H_5)SiO_{2/2}$.

L'organopolysiloxane **B** peut présenter une structure linéaire, ramifiée, cyclique ou en réseau.

Lorsqu'il s'agit de polyorganosiloxanes linéaires, ceux-ci peuvent être essentiellement constitués :

- de motifs siloxyles « D » choisi parmi les motifs de formules $(H)(L)SiO_{2/2}$ et $(L)_2SiO_{2/2}$ ;
- de motifs siloxyles « M » choisis parmi les motifs de formules $(H)(L)_2SiO_{1/2}$ et $(L)_3SiO_{2/2}$,
- avec le symbole L ayant la même signification que ci-dessus et le symbole H désignant un atome d'hydrogène.

Ces polyorganosiloxanes linéaires peuvent être des huiles ayant une viscosité dynamique à 25°C comprise entre 1 mPa.s et 5000 mPa.s, préférentiellement entre 1 mPa.s et 1000 mPa.s, et encore plus préférentiellement entre 1 mPa.s et 500 mPa.s.

Lorsqu'il s'agit de polyorganosiloxanes cycliques, ceux-ci peuvent être constitués de motifs siloxyles « D » choisi parmi les motifs de formules $HLSiO_{2/2}$ et $L_2SiO_{2/2}$, ou de motifs siloxyle de formule $HLSiO_{2/2}$ uniquement. Les motifs de formule $L_2SiO_{2/2}$ peuvent être notamment des dialkylsiloxy ou des alkylarylsiloxy. Ces polyorganosiloxanes cycliques peuvent avoir une viscosité dynamique à 25°C comprise entre 1 mPa.s et 5 000 mPa.s.

Des exemples d'organopolysiloxane **B** sont :

- les polydiméthylsiloxanes à extrémités hydrogénodiméthylsilyles ;
- les poly(diméthylsiloxane-co-hydrogénométhylsiloxane) à extrémités triméthylsilyles ;
- les poly(diméthylsiloxane-co-hydrogénométhylsiloxane) à extrémités hydrogénodiméthyl-silyles ;
- les polyhydrogénométhylsiloxanes à extrémités triméthylsilyles ;
- les hydrogénométhylpolysiloxanes cycliques.

Lorsqu'il s'agit de polyorganosiloxanes ramifiés ou en réseaux, ceux-ci peuvent comprendre en outre :

- des motifs siloxyles « T » choisis parmi les motifs de formules $(H)SiO_{3/2}$ et $(L)SiO_{3/2}$ ;
- des motifs siloxyles « Q » de formule $SiO_{4/2}$,
- avec le symbole H représentant un atome d'hydrogène et L ayant la même signification que ci-dessus.

Selon un mode de réalisation particulièrement avantageux, la composition silicone **X** comprend au moins deux organopolysiloxanes **B** différents qui sont :

a) un allongeur de chaine **B**$^{all}$ comprenant :

- des motifs siloxy monovalent terminaux, identiques ou différents, de formule (M) :

$$(H)_p(R^1)_qSiO_{1/2} \qquad (M)$$

dans laquelle le symbole $R^1$ correspond à un groupe alkyle en $C_1$ à $C_8$; le symbole H représente un atome d'hydrogène et avec p= 0 ou 1, q=2 ou 3 et (p+q)= 3;
- des motifs siloxy bivalent, identiques ou différents, de formule (D) :

$$(H)_n(R^2)_mSiO_{2/2} \qquad (D)$$

dans laquelle le radical $R^2$ correspond à un groupe alkyle en $C_1$ à $C_8$ ou un groupe aryle, le symbole H représente un atome d'hydrogène et avec n= 0 ou 1, m =1 ou 2 et (n+m)= 2, et
- avec la condition selon laquelle l'organopolysiloxanes **B**$^{\mathbf{all}}$ comprend deux atomes d'hydrogène liés chacun à un atome de silicium différent par polymère, c'est-à-dire deux fonctions Si-H par polymère ;

b) un réticulant **B**$^{\mathbf{ret}}$ comprenant :

- au moins trois motifs siloxy de formule (B.1) :

$$(H)\ (L)_e\ SiO_{(3-e)/2} \qquad (B.1)$$

dans laquelle le symbole H représente un atome d'hydrogène, le symbole L représente un alkyle ayant de 1 à 8 atomes de carbone inclus ou un aryle tel que xylyle, tolyle ou phényle, et le symbole e est égal à 0, 1 ou 2 ; et

- éventuellement des autres motifs siloxy de formule (B-2) :

(L)g SiO(4-g)/2

dans laquelle le symbole L représente un alkyle ayant de 1 à 8 atomes de carbone inclus et le symbole g est égal à 0, 1, 2 ou 3.

Ce mode de réalisation mettant un œuvre un mélange d'organopolysiloxane **B** jouant le rôle d'allongeur de chaîne (**B**$^{\mathbf{all}}$) et d'organopolysiloxane jouant le rôle de réticulant **B**$^{\mathbf{ret}}$ permet d'obtenir des gels ayant des propriétés d'adhésion améliorée, notamment vis-à-vis du support **S** employé. L'homme du métier suivant le type de support utilisé sera faire varier les teneurs en poids de chaque organopolysiloxane jouant le rôle d'allongeur de chaîne (**B**$^{\mathbf{all}}$) et d'organopolysiloxane **A** jouant le rôle de réticulant **B**$^{\mathbf{ret}}$**.** Par exemple il pourra se référer à l'enseignement décrit dans le brevet EP-0737721-B1 déposé par la société Rhodia Chimie.

Des organopolysiloxanes allongeur de chaine **B**$^{\mathbf{all}}$ particulièrement avantageux sont les polydiméthylsiloxanes à extrémités diméthylhydrogénosilyles ayant une viscosité dynamique à 25°C comprise entre 1 mPa.s et 500 mPa.s, de préférence comprise entre 1 mPa.s et 250 mPa.s, et encore plus préférentiellement comprise entre 1 et 50 mPa.s. La formule de ces organopolysiloxanes **B**$^{\mathbf{al}}$ particulièrement avantageux est $M^HD_xM^H$, formule dans laquelle :

- $M^H$ = motif siloxy de formule : $(H)(CH_3)_2SiO_{1/2}$
- D = motif siloxyl de formule : $(CH_3)_2SiO_{2/2}$, et

x est un nombre compris entre 0 et 100, et de préférence compris entre 1 et 50, et encore plus préférentiellement compris entre 3 et 30.

Des organopolysiloxanes réticulant **B**$^{\mathbf{ret}}$ particulièrement avantageux sont les polydiméthylsiloxanes à extrémités diméthylhydrogénosilyles ayant une viscosité dynamique à 25°C comprise entre 1 mPa.s et 2000 mPa.s, de préférence comprise entre 1 mPa.s et 1000 mPa.s, et encore plus préférentiellement comprise entre 1 et 500 mPa.s. Les formules de ces organopolysiloxanes **B**$^{\mathbf{al}}$ particulièrement avantageux sont :

- $M^HD_xD_w{}^HM^H$
- $M^HD_xD_y{}^HM$
- $M\ D_x\ Dz^H\ M$

formules dans lesquelles :

- $M^H$ = motif siloxy de formule : $(H)(CH_3)_2SiO_{1/2}$
- $D^H$ = motif siloxy de formule : $(H)(CH_3)SiO_{2/2}$
- D = motif siloxyl de formule : $(CH_3)_2SiO_{2/2}$; et
- M = motif siloxy de formule : $(CH_3)_3SiO_{1/2}$

  - avec :

    - x est un nombre compris entre 0 et 500, de préférence entre 10 et 250, et encore plus préférentiellement entre 50 et 150,
    - w est un nombre compris entre 1 et 500, de préférence compris entre 1 et 250 ou entre 1 et 100, et encore plus préférentiellement compris entre 1 et 20 ;
    - y est un nombre compris entre 2 et 500, de préférence compris entre 2 et 250 ou entre 2 et 100, et encore plus préférentiellement compris entre 2 et 20 ; et
    - z est un nombre compris entre 3 et 500, de préférence compris entre 3 et 250 ou entre 3 et 100, et encore plus préférentiellement compris entre 3 et 20.

Selon un mode de réalisation préféré, le choix et la quantité de l'organopolysiloxanes allongeur de chaine $\mathbf{B^{all}}$ et de l'organopolysiloxane réticulant $\mathbf{B^{ret}}$ est fait de manière à ce que le ratio ri décrit ci-dessous soit inférieur ou égal à 80 % :

$$r_1 = \frac{\text{Nombre de mole de fonction SiH de l'allongeur } B^{all}}{\text{Nombre de mole total de fonction SiH (allongeur } B^{all} + \text{réticulant } B^{ret})} \times 100$$

La résine silicone **Z** est un polymère organopolysiloxane ramifié comprenant des motifs siloxy Q ($SiO_{4/2}$) est bien connue et est disponible dans le commerce. Elle est mise en œuvre sous forme diluée de préférence dans une huile silicone qui peut porter des fonctions vinylées ou un mélange huile silicone et gomme silicone (telle que décrite dans le présent mémoire). Dans ce cas, le choix de l'huile silicone et/ou de la gomme se fera de manière à avoir un mélange ayant une viscosité dynamique à 25°C (de la forme diluée) comprise entre 1000 mPa.s et 100000 mPa.s.

Selon une autre variante, la résine silicone Z est introduite sous la la forme d'un mélange de cette résine dans une gomme silicone.

La résine silicone **Z** particulièrement utile selon l'invention est une résine silicone ayant au moins un motif siloxy Q et qui comporte dans sa structure de 0,1 à 20 % en poids de groupe(s) alcényle(s). Dans cette résine, les groupements alcényles peuvent être situés sur des motifs siloxyles M, D ou T. Ces résines peuvent être préparées par exemple selon le procédé décrit dans le brevet US -A- 2 676 182. Un certain nombre de ces résines sont disponibles dans le commerce, le plus souvent à l'état de solutions, par exemple dans du xylène.

Dans un mode préféré de réalisation de l'invention, la résine silicone **Z** est choisie parmi le groupe constitué par les résines silicones de formules suivantes :

- $MD^{Vi}Q$ où les groupes vinyles sont inclus dans les motifs D,
- $MD^{Vi}TQ$ où les groupes vinyles sont inclus dans les motifs D,
- $MM^{Vi}Q$ où les groupes vinyles sont inclus dans une partie des motifs M,
- $MM^{Vi}TQ$ où les groupes vinyles sont inclus dans une partie des motifs M,
- $MM^{Vi}DD^{Vi}Q$ où les groupes vinyles sont inclus dans les motifs M et D,
- et leurs mélanges,

avec :

- M = motif siloxyle de formule $R_3SiO_{1/2}$
- $M^{Vi}$ = motif siloxyle de formule $(R_2)(vinyle)SiO_{1/2}$
- D = motif siloxyle de formule $R_2SiO_{2/2}$
- $D^{Vi}$ = motif siloxyle de formule $(R)(vinyle)SiO_{2/2}$

- Q = motif siloxyle de formule $SiO_{4/2}$ ;
- T = motif siloxyle de formule $RSiO_{3/2}$, et
- les groupements R, identiques ou différents, sont des groupes hydrocarbonés monovalents choisis parmi les groupes alkyles ayant de 1 à 8 atomes de carbone inclus tels que les groupes méthyle, éthyle, propyle et 3,3,3-trifluoropropyle et les groupes aryle tels que xylyle, tolyle et phényle. De préférence, les groupements R sont des méthyles.

Selon un autre mode particulier de l'invention, la résine silicone **Z** est additionnée à la composition selon l'invention sous forme d'un mélange dans au moins une huile organopolysiloxane, par exemple correspondant à la définition de l'organopolysiloxane **A** décrit ci-dessus, ou dans au moins un solvant hydrocarboné tels que le toluène, le xylène ou des dérivés connus sous l'appellation Exxsol® et vendus par la société Exxon Mobil.

De préférence la résine silicone **Z** ayant des groupements alcényles liés à des atomes de silicium est choisie parmi le groupe constitué par :

- une résine silicone $\mathbf{Z^1}$ de formule $MD^{vi}Q$ dans laquelle :
  - M est un motif siloxy de formule $R_3SiO_{1/2}$ dans laquelle R est un alkyle en $C_1$ à $C_8$ ou un groupe aryle tel que xylyle, tolyle ou phényle,
  - $D^{Vi}$ est un motif siloxy de formule $RR^1SiO_{2/2}$ avec R étant un groupe alkyle en $C_1$ à $C_8$ ou un groupe aryle et $R^1$ étant un groupe vinyle, et
  - Q est un motif siloxy de formule $SiO_{4/2}$ ;
- une résine silicone $\mathbf{Z^2}$ de formule $MM^{Vi}Q$ dans laquelle :
  - M est un motif siloxy de formule $R_3SiO_{1/2}$ dans laquelle R est un alkyle en $C_1$ à $C_8$ ou un groupe aryle tel que xylyle, tolyle ou phényle,
  - $M^{Vi}$ est un motif siloxy de formule $R_2(Vi)SiO_{1/2}$ avec Vi étant un groupe vinyle et R étant un groupe alkyle en $C_1$ à $C_8$ ou un groupe aryle tel que xylyle, tolyle ou phényle, et
  - Q est un motif siloxy de formule $SiO_{4/2}$, et
- les mélanges des résines silicones $\mathbf{Z^1}$ et $\mathbf{Z^2}$.

Comme catalyseur **C** d'hydrosilylation utile selon l'invention, on peut citer les composés d'un métal appartenant au groupe du platine bien connu de l'homme de l'art. Les métaux du groupe du platine sont ceux connus sous le nom de platinoïdes, appellation qui regroupe, outre le platine, le ruthénium, le rhodium, le palladium, l'osmium et l'iridium. On utilise, de préférence, les composés du platine et du rhodium. On peut, en particulier, utiliser les complexes du platine et d'un produit organique décrit dans les brevets US-A-3 159 601, US-A-3 159 602, US-A-3 220 972 et les brevets européens EP-A-0 057 459, EP-A-0 188 978 et EP-A-0 190 530, les complexes du platine et d'organosiloxanes vinylés décrits dans les brevets US-A-3 419 593. Le catalyseur généralement préféré est le platine. A titre d'exemples, on peut citer le platine noir, l'acide chloroplatinique, un acide chloroplatinique modifié par un alcool, un complexe de l'acide chloroplatinique avec une oléfine, un aldéhyde, un vinylsiloxane ou un alcool acétylénique, entre autres. La préférence va à la solution ou complexe de Karstedt, tel que décrit dans le brevet US-A-3 775 452, à l'acide chloroplatinique hexahydrate ou un catalyseur au platine comprenant des ligands carbènes.

Comme inhibiteur **D** de la réaction d'hydrosilylation utile selon l'invention, on peut citer celui choisi parmi les alcools $\alpha$-acétyléniques, les diesters $\alpha$-$\alpha$'-acétyléniques, les composés conjugués ène-yne, les cétones $\alpha$-acétyléniques, les acrylonitriles, les maléates, les fumarates et les mélanges de ceux-ci. Ces composés capables de remplir la fonction d'inhibiteur d'hydrosilylation sont bien connus de l'homme du métier. Ils peuvent être utilisés seuls ou en mélanges.

Un inhibiteur **D** de type alcool $\alpha$-acétylénique peut être choisi parmi les composés de formule (D1) suivante :

$$(R^1)(R^2)C(OH)\text{-}C{:}CH \qquad (D1)$$

dans laquelle :

- le groupe $R^1$ représente un groupe alkyle, un groupe cycloalkyle, un groupe (cycloalkyl)alkyle, un groupe aryle ou un groupe arylalkyle,
- le groupe $R^2$ représente un atome d'hydrogène, un groupe alkyle, un groupe cycloalkyle, un groupe (cycloalkyl)alkyle, un groupe aryle ou un groupe arylalkyle,
- ou bien $R^1$ et $R^2$ constituent ensemble avec l'atome de carbone auquel ils sont liés un cycle aliphatique à 5, 6, 7 ou 8 chaînons, éventuellement substitué une ou plusieurs fois.

Selon la formule (D1) :

- Par « alkyle », on entend une chaîne hydrocarbonée saturée contenant de 1 à 20 atomes de carbone, de préférence de 1 à 8 atomes de carbone. Un groupe alkyle peut être choisi dans le groupe constitué par méthyle, éthyle, isopropyle, n-propyle, tert-butyle, isobutyle, n-butyle, n-pentyle, isoamyle et 1,1-diméthylpropyle ;
- Par « cycloalkyle », on entend selon l'invention un groupe hydrocarboné saturé monocyclique ou polycyclique, de préférence monocyclique ou bi cyclique, contenant de 3 à 20 atomes de carbone, de préférence de 5 à 8 atomes de carbone. Lorsque le groupe cycloalkyle est polycyclique, les multiples noyaux cycliques peuvent être rattachés les uns aux autres par une liaison covalente et/ou par un atome spinanique et/ou être condensés les uns aux autres. Un groupe cycloalkyle peut être choisi dans le groupe constitué par le cyclopropyle, le cyclobutyle, le cyclopentyle, le cyclohexyle, le cycloheptyle, le cyclooctyle, l'adamantane et le norborane ;
- Par « (cycloalkyl)alkyle », on entend selon l'invention un groupe cycloalkyle tel que défini ci-avant lié à un groupe alkyle tel que défini ci-avant également.
- Par « aryle », on entend selon l'invention un groupe hydrocarboné aromatique contenant de 5 à 18 atomes de carbone, monocyclique ou polycyclique. Un groupe aryle peut être choisi dans le groupe constitué par phényle, naphtyle, anthracényle et phénanthryle ;
- Par « arylalkyle », on entend selon l'invention un groupe aryle tel que défini ci-avant lié à un groupe alkyle tel que défini ci-avant également.

Selon un mode de réalisation préféré, dans la formule (D1) $R^1$ et $R^2$ constituent ensemble avec l'atome de carbone auquel ils sont liés un cycle aliphatique non substitué à 5, 6, 7 ou 8 chaînons. Selon un autre mode de réalisation préféré, $R^1$ et $R^2$, identiques ou différents, représentent indépendamment l'un de l'autre un groupe alkyle monovalent en $C_1$ à $C_{12}$, de préférence en $C_1$ à $C_6$.

Un inhibiteur D qui est un alcool $\alpha$-acétylénique utile selon l'invention peut être choisi dans le groupe constitué par les composés suivants : 1-éthynyl-1-cyclopentanol ; 1-éthynyl-1-cyclohexanol (aussi appelé ECH) ; 1-éthynyl-1-cycloheptanol ; 1-éthynyl-1-cyclooctanol ; 3-méthyl-1-butyn-3-ol (aussi appelé MBT) ; 3-méthyl-1-pentyn-3-ol ; 3-méthyl-1-hexyn-3-ol ; 3-méthyl-1-heptyn-3-ol ; 3-méthyl-1-octyn-3-ol ; 3-méthyl-1-nonyn-3-ol ; 3-méthyl-1-decyn-3-ol ; 3-méthyl-1-dodecyn-3-ol ; 3-méthyl-1-pentadecyn-3-ol ; 3-éthyl-1-pentyn-3-ol ; 3-éthyl-1-hexyn-3-ol ; 3-éthyl-1-heptyn-3-ol ; 3,5-diméthyl-1-hexyn-3-ol ; 3-isobutyl-5-méthyl-1-hexyn-3-ol ; 3,4,4-triméthyl-1-pentyn-3-ol ; 3-éthyl-5-méthyl-1-heptyn-3-ol ; 3,6-diéthyl-1-nonyn-3-ol ; 3,7,11-triméthyl-1-dodecyn-3-ol (aussi appelé TMDDO) ; 1,1-diphényl-2-propyn-1-ol ; 3-butyn-2-ol ; 1-pentyn-3-ol ; 1-hexyn-3-ol ; 1-heptyn-3-ol ; 5-méthyl-1-hexyn-3-ol ; 4-éthyl-1-octyn-3-ol et 9-éthynyl-9-fluorenol.

Un inhibiteur D de type diester $\alpha$-$\alpha$'-acétylénique peut être choisi parmi les composés de formule (D2) suivante :

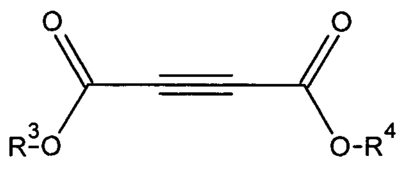

(D2)

dans laquelle les groupe $R^3$ et $R^4$, identiques ou différents, représentent indépendamment l'un de l'autre un groupe alkyle, un groupe cycloalkyle, un groupe (cycloalkyl)alkyle, un groupe aryle, un groupe arylalkyle ou un groupe silyle.

Par « silyle », on entend selon l'invention un groupe de formule $-SiR_3$, chaque R représentant indépendamment un groupe alkyle contenant de 1 à 20 atomes de carbone, de préférence de 1 à 8 atomes de carbone. Un groupe silyle peut être par exemple le groupe triméthylsilyle.

Selon un mode de réalisation particulier, dans la formule (D2) $R^3$ et $R^4$, identiques ou différents, représentent indépendamment l'un de l'autre un groupe alkyle en $C_1$ à $C_{12}$, de préférence en $C_1$ à $C_6$, ou le groupe triméthylsilyle. Un inhibiteur **D** qui est un diester $\alpha$-$\alpha$'-acétylénique utile selon l'invention peut être choisi dans le groupe constitué par les composés suivants : l'acétylène-dicarboxylate de diméthyle (DMAD), l'acétylène-dicarboxylate de diéthyle, l'acétylène-dicarboxylate de tert-butyle et l'acétylène-dicarboxylate de bis(triméthylsilyle).

Un inhibiteur **D** de type composé conjugué ène-yne peut être choisi parmi les composés de formule (D3) suivante :

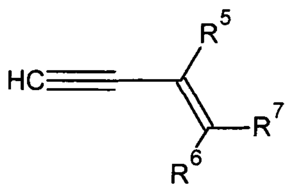

(D3)

dans laquelle :

- les groupes $R^5$, $R^6$ et $R^7$ représentent, indépendamment les uns des autres, un atome d'hydrogène, un groupe alkyle, un groupe cycloalkyle, un groupe (cycloalkyl)alkyle, un groupe aryle ou un groupe arylalkyle,
- ou bien au moins deux groupes parmi les groupes $R^5$, $R^6$ et $R^7$ constituent ensemble avec le ou les atomes de carbone auxquels ils sont liés un cycle aliphatique à 5, 6, 7 ou 8 chaînons, éventuellement substitué une ou plusieurs fois.

Selon un mode de réalisation particulier, les groupes $R^5$, $R^6$ et $R^7$ représentent, indépendamment les uns des autres, un atome d'hydrogène, un groupe alkyle en $C_1$ à $C_{12}$, de préférence en $C_1$ à $C_6$, ou un groupe aryle. Un inhibiteur **D** qui est un composé conjugué ène-yne utile selon l'invention peut être choisi dans le groupe constitué par les composés suivants : le 3-méthyl-3-pentène-1-yne ; le 3-méthyl-3-hexène-1-yne ; le 2,5-diméthyl-3-hexène-1-yne ; le 3-éthyl-3-butène-1-yne ; et le 3-phényl-3-butène-1-yne. Selon un autre mode de réalisation particulier, deux groupes choisis parmi les groupes $R^5$, $R^6$ et $R^7$ constituent ensemble avec le ou les atomes de carbone auxquels ils sont liés un cycle aliphatique non substitué à 5, 6, 7 ou 8 chaînons et le troisième groupe restant représente un atome d'hydrogène ou un groupe alkyle en $C_1$ à $C_{12}$, de préférence en $C_1$ à $C_6$. Un inhibiteur **D** qui est un composé conjugué ène-yne utile selon l'invention peut être le 1-éthynyl-1-cyclohexène.

Un inhibiteur **D** de type cétone $\alpha$-acétylénique peut être choisi parmi les composés de formule (D4) suivante :

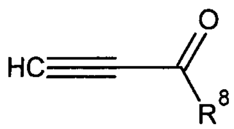

(D4)

dans laquelle : $R^8$ représente un groupe alkyle, un groupe cycloalkyle, un groupe (cycloalkyl)alkyle, un groupe aryle ou un groupe arylalkyle, les groupes alkyles, cycloalkyles, (cycloalkyl)alkyle, aryle ou arylalkyle pouvant éventuellement être substitué une ou plusieurs fois par un atome de chlore, de brome ou d'iode.

Selon un mode de réalisation préféré, $R^8$ représente un groupe alkyle monovalent en $C_1$ à $C_{12}$, de préférence en $C_1$ à $C_6$, éventuellement être substitué une ou plusieurs fois par un atome de chlore ou de brome, ou un groupe cycloalkyle, ou un groupe aryle. Un inhibiteur **D** qui est une cétone $\alpha$-acétylénique utile selon l'invention peut être choisi dans le groupe constitué par les composés suivants : la 1-octyn-3-one, la 8-chloro-1-octyn-3-one ; la 8-bromo-1-octyn-3-one ; la 4,4-diméthyl-1-octyn-3-one ; la 7-chloro-1-heptyn-3-one ; la 1-hexyn-3-one ; la 1-pentyn-3-one ; la 4-méthyl-1-pentyn-3-one ; la 4,4-diméthyl-1-pentyn-3-one ; la 1-cyclohexyl-1-propyn-3-one ; le benzoacétylène et le o-chlorobenzoyl-acétylène.

Un inhibiteur D de type acrylonitrile peut être choisi parmi les composés de formule (D5) suivante :

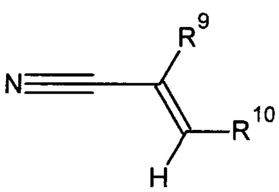

(D5)

dans laquelle : $R^9$ et $R^{10}$ représentent, indépendamment l'un de l'autre, un atome d'hydrogène, un atome de chlore, de brome ou d'iode, un groupe alkyle, un groupe cycloalkyle, un groupe (cycloalkyl)alkyle, un groupe aryle ou un groupe arylalkyle, les groupes alkyles, cycloalkyles, (cycloalkyl)alkyle, aryle ou arylalkyle pouvant éventuellement être substitué une ou plusieurs fois par un atome de chlore, de brome ou d'iode.

Un inhibiteur **D** qui est un acrylonitrile utile selon l'invention peut être choisi dans le groupe constitué par les

composés suivants : l'acrylonitrile ; le méthacrylonitrile ; le 2-chloroacrylonitryle ; le crotononitrile et le cinnamonitrile.

Un inhibiteur **D** de type maléate ou fumarate peut être choisi parmi les composés de formules (D6) et (D7) suivantes :

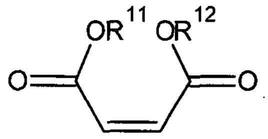
(D6)

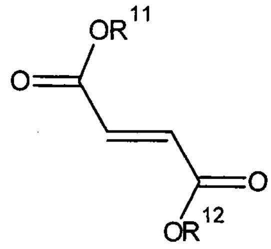
(D7)

dans lesquelles : $R^{11}$ et $R^{12}$, identiques ou différents, représentent indépendamment l'un de l'autre un groupe alkyle ou alcényle, un groupe cycloalkyle, un groupe (cycloalkyl)alkyle, un groupe aryle ou un groupe arylalkyle, lesdits groupes alkyles, alcényles, cycloalkyles, (cycloalkyl)alkyles, aryles et arylalkyles pouvant être substitués par un groupe alcoxy

Par le terme « alcényle », on entend selon l'invention une chaîne hydrocarbonée saturée contenant de 1 à 20 atomes de carbone, de préférence de 1 à 6 atomes de carbone et comprenant au moins une double insaturation. De préférence, le groupe alcényle est choisi dans le groupe constitué par un vinyle ou un allyle.

Par « alcoxy », on entend selon les formules (D6) ou (D7), un groupe alkyle tel que défini ci-avant lié à un atome d'oxygène. Un groupe alcoxy peut être choisi dans le groupe constitué par méthoxy, éthoxy, propoxy et butoxy.

Selon un mode de réalisation particulier, $R^{11}$ et $R^{12}$, identiques ou différents, représentent indépendamment l'un de l'autre un groupe alkyle ou alcényle en $C_1$ à $C_{12}$, de préférence en $C_1$ à $C_6$, éventuellement substitué par un groupe alcoxy en $C_1$ à $C_6$.

Un inhibiteur **D** qui est un maléate ou un fumarate utile selon l'invention peut être choisi dans le groupe constitué par le fumarate de diéthyle, le maléate de diéthyle, le fumarate de diallyle, le maléate de diallyle et le maléate de bis-(méthoxyisopropyle).

Des inhibiteurs **D** choisis parmi les alcools $\alpha$-acétyléniques, les diesters $\alpha$-$\alpha$'-acétyléniques, les composés conjugués ène-yne, les cétones $\alpha$-acétyléniques, les acrylonitriles, les maléates, les fumarates sont disponibles dans le commerce. On peut citer notamment l'ECH (1-éthynyl-1-cyclohexanol) qui est disponible commercialement chez BASF, le maléate de diméthyle qui est disponible commercialement chez DMS et l'acétylène-dicarboxylate de diméthyle qui est disponible chez City Chemical LLC.

Ces inhibiteurs sont ajoutés en quantité en poids comprise entre 1 et 50 000 ppm par rapport au poids de la composition silicone totale, notamment entre 10 et 10 000 ppm, de préférence entre 20 et 2000 ppm et encore plus préférentiellement entre 20 ppm et 500 ppm.

Comme exemple d'additif stabilisant **K** on peut par exemple citer des dérivés silylés de l'acide phosphorique tels que les esters silylés de l'acide phosphorique.

Des résultats particulièrement avantageux sont obtenus lorsque la composition silicone X comprend:

1) au moins un organopolysiloxane **A** qui est un polydiméthylsiloxane à extrémités diméthylvinylsilyles ayant une viscosité dynamique à 25°C comprise entre 50 mPa.s et 120 000 mPa.s, et de préférence comprise entre 100 mPa.s et 80000 mPa.s et ayant une formule $M^{Vi}D_xM^{Vi}$, formule dans laquelle :

- $M^{Vi}$ = motif siloxy de formule : (vinyle)$(CH_3)_2SiO_{1/2}$
- D = motif siloxyl de formule : $(CH_3)_2SiO_{2/2}$, et
- x est un nombre compris entre 0 et 1000, et de préférence compris entre 5 et 1000

2) un organopolysiloxane allongeur de chaine $\mathbf{B^{all}}$ de formule $M^HD_xM^H$ avec :

- $M^H$ = motif siloxy de formule : $(H)(CH_3)_2SiO_{1/2}$
- D = motif siloxy de formule : $(CH_3)_2SiO_{2/2}$, et
  et x étant un nombre compris entre 3 et 30,

3) un organopolysiloxane réticulant $\mathbf{B^{ret}}$ de formule $M^H\ D_x\ D_w{}^H\ M^H$ ou $M^H\ D_x\ D_y{}^H\ M$ , formules dans lesquelles :

- $M^H$ = motif siloxy de formule : $(H)(CH_3)_2SiO_{1/2}$
- M = motif siloxy de formule : $(CH_3)_3SiO_{1/2}$

- D = motif siloxy de formule : $(CH_3)_2SiO_{2/2}$, et
- $D^H$ = motif siloxyl de formule : $(CH_3)(H)\ SiO_{2/2}$, et

    ◦ x est un nombre compris entre 50 et 150,
    ◦ w est un nombre compris entre 1 et 20 ; et
    ◦ y est un nombre compris entre 2 et 20 ;

4) au moins un catalyseur **C** d'hydrosilylation,
5) au moins un inhibiteur **D** de la réaction d'hydrosilylation,
6) éventuellement au moins un additif **K,** et
7) entre 1,5 % et 3,5 % en poids, et de préférence entre 1,75% et 3,0 % en poids, par rapport au poids total de la composition silicone **X** d'au moins une résine silicone $\mathbf{Z^1}$ de formule $MD^{vi}Q$ et telle que décrite ci-dessus et/ou d'au moins une résine silicone $\mathbf{Z^2}$ de formule $MM^{Vi}Q$ et telle que décrite ci-dessus, et
avec les conditions suivantes :

a) les quantités en poids des organopolysiloxanes **A**, **B,** $\mathbf{Z^1}$ et $\mathbf{Z^2}$ sont déterminées de manière à ce que la valeur du rapport $RH_a ik = n_H/tAlk$ soit comprise dans l'intervalle suivant : $0,10 \leq RH_{alk} \leq 0,80$, de préférence dans l'intervalle suivant $0,20 \leq RH_{alk} \leq 0,80$, et encore plus préférentiellement dans l'intervalle suivant $0,20 \leq RH_{alk} \leq 0,75$, avec $n_H$ = nombre de mole d'atome d'hydrogène directement lié à un atome de silicium des organopolysiloxanes $\mathbf{B^{all}}$ et $\mathbf{B^{ret}}$ et $t_{Alk}$ = nombre de mole d'alcényle directement lié à un atome de silicium de l'organopolysiloxane **A** et de la résine silicone $\mathbf{Z^1}$ ou $\mathbf{Z^2}$ ou du mélange de résine $\mathbf{Z^1}$ et $\mathbf{Z^2}$**,** et
b) les viscosités et les quantités en poids des constituants de la composition silicone **X** sont choisies de manière à ce que la viscosité dynamique à 25°C de la composition silicone **X** soit comprise entre 200 mPa.s et 100000 mPa.s, et de préférence comprise entre 200 mPa.s et 80000 mPa.s.

Selon un mode de réalisation particulièrement avantageux, la composition silicone **X** comprend comme organopolysiloxane **A** au moins deux organopolysiloxanes **A1** et **A2** comportant, par molécule, au moins deux radicaux alcényles en $C_2$ à $C_6$ liée chacun à un atome de silicium, caractérisé en ce :

a) l'organopolysiloxanes **A1** à une viscosité dynamique à 25°C comprise entre 100 mPa.s et 120000 mPa.s, et
b) l'organopolysiloxanes **A2** est une gomme ayant une consistance à 25°C comprise entre 500 mm/10 et 1000 mm/10.

De préférence les deux organopolysiloxanes **A1** et **A2** dont des polydiméthylsiloxanes à extrémités diméthylvinylsilyles et caractérisé en ce :

a) l'organopolysiloxanes **A1** à une viscosité dynamique à 25°C comprise entre 100 mPa.s et 120000 mPa.s, et
b) l'organopolysiloxanes **A2** est une gomme ayant une consistance à 25°C comprise entre 500 mm/10 et 1000 mm/10.

Le terme « gomme » est utilisé pour des composés organosiliciques présentant des viscosités classiquement supérieures à ~600000 mPa.s ce qui correspond à un poids moléculaire supérieur à 260000 g/mole. La consistance d'une gomme est déterminée à 25°C au moyen d'un pénétromètre de type PNR12 ou modèle équivalent permettant d'appliquer sur l'échantillon une tête cylindrique dans des conditions normalisées. La consistance d'une gomme est la profondeur exprimée en dixième de millimètres à laquelle un cylindre calibré pénètre dans l'échantillon pendant une minute. A cet effet, un échantillon de gomme est introduit dans un godet en aluminium de diamètre 40 mm et d'une hauteur de 60 mm. La tête cylindrique en bronze ou en laiton mesure 6,35 mm de diamètre et 4,76 mm de hauteur et est portée par une tige métallique de 51 mm de long et de 3mm de diamètre qui s'adapte au pénétromètre. Cette tige est lestée d'une surcharge de 100 g. Le poids total de l'ensemble est de 151,8 g. Le godet contenant l'échantillon de gomme est mis dans le bain thermostaté à 25°C ± 0,5 ° pendant au minimum 30 minutes. La mesure est effectuée en suivant la notice du constructeur.

Selon une variante préférée, la composition silicone **X** comprend au moins trois organopolysiloxanes **A** tels que décrits ci-dessus et comportant, par molécule, au moins deux radicaux alcényles en $C_2$ à $C_6$ liés chacun à un atome de silicium :

a) le premier ayant une viscosité comprise entre 100 mPa.s et 5000 mPa.s,
b) le deuxième ayant une viscosité comprise entre 5000 mPa.s et 15000 mPa.s, et
c) le troisième ayant une viscosité comprise entre 15000 mPa.s et 100000 mPa.s.

Le dernier objet selon l'invention concerne un article adhésif à la peau caractérisé en ce qu'il s'agit d'un dispositif

médical ou d'un élément d'un dispositif médical.

Comme exemples de dispositifs médicaux, on peut citer un pansement adhésif à la peau en particulier destinés au retrait atraumatique d'une peau saine et d'une plaie, ou un dispositif de maintien d'accessoires médicaux utilisés au contact de la peau de type capteur, sonde, cathéter ou aiguille.

Les exemples non limitatifs qui suivent, montrent diverses possibilités de formulation des compositions selon l'invention ainsi que les caractéristiques et les propriétés des gels silicones obtenus par réticulation desdites compositions.

**EXEMPLES**

1) Mesure du collant instantané ou « tack » :

Le test est réalisé selon la norme ASTM D2979 avec un appareil PROBE TACK Device (PT-1000). Un poinçon cylindrique à face plane est amené en contact avec le gel du composite à tester (surface de contact avec le gel= $0.2cm^2$). Le composite est constitué d'un support en PET (épaisseur de 36 $\mu m$) enduit à 200 $g/m^2$ de la composition silicone précurseur du gel. Le poinçon est maintenu ensuite en contact avec le gel pendant un temps de contact de 1 seconde à une pression constante de 100 $gf/cm^2$. Ensuite, le poinçon est décollé à vitesse constante de 10 mm/s du gel, et la force nécessaire pour séparer le gel de la tige est mesurée et s'exprime en $gf/cm^2$.

2) Le pouvoir adhésif à 180° ou test de pelage à 180° (« peel » en anglais)

Le composite est constitué d'un support en PET (épaisseur de 36 $\mu m$) enduit à 200 $g/m^2$ de la composition silicone précurseur du gel. Le pouvoir adhésif à 180° est évalué par la méthodologie décrite dans le document FINAT Test Method n°1 (FINAT Technical Handbook 6th Edition, 2001). Ainsi, le gel silicone du composite à tester (dimensions = 15 cm de longueur et 4 cm de largeur) est mis en contact avec une feuille de papier bristol (marque Exacompta®). Le composite est ensuite décollé avec un angle de 180° avec une vitesse constante de 300mm/min et avec l'aide d'une cellule de force de 10N. La force est mesurée et ramenée à la largeur de l'article et s'exprime en N/cm.

3) Résistance au cisaillement statique (Shear)

Le composite est constitué d'un support en PET (épaisseur de 36 $\mu m$) enduit à 200 $g/m^2$ de la composition silicone précurseur du gel.

La résistance au cisaillement statique ou « shear »est évaluée par la méthodologie décrite dans le document « FINAT Test Method no. 8 » (FINAT Technical Handbook 6th Edition, 2001).

Le composite (dimensions : 4.5 cm x 2.5 cm) est collée à une plaque métallique (acier-inox) et est soumis à un poids de 1kg. Le temps nécessaire jusqu'à la chute du poids est mesuré en heure et correspond au « shear ».

4) Calcul du ratio r1

$$r_1 = \frac{\text{Nombre de mole de fonction SiH de l'allongeur } B^{all}}{\text{Nombre de mole total de fonction SiH (allongeur } B^{all} + \text{réticulant } B^{ret}\text{)}} \times 100$$

5) Préparation des compositions silicones précurseurs de gel selon l'invention

a) Matières premières utilisées

- POS **A1** = huile polydiméthylsiloxane $\alpha$, $\omega$(diméthylvinylsiloxy) ayant une viscosité dynamique à 25°C égale à 60000 mPa.s.
- POS **A2** = huile polydiméthylsiloxane $\alpha$, $\omega$(diméthylvinylsiloxy) ayant une viscosité dynamique à 25°C égale à 10000 mPa.s.
- POS **A3** = huile polydiméthylsiloxane $\alpha$, $\omega$(diméthylvinylsiloxy) ayant une viscosité dynamique à 25°C égale à 3500

mPa.s.

- Résine silicone **Z¹** : de formule $MD^{vi}Q$ dans laquelle :
    - M est un motif siloxy de formule : $(CH_3)_3SiO_{1/2}$ ;
    - $D^{Vi}$ est un motif siloxy de formule $(CH_3)(vinyle)SiO_{2/2}$
    - Q est un motif siloxy de formule $SiO_{4/2}$
- Résine silicone **Z²** de formule $MM^{Vi}Q$ dans laquelle:
    - M est un motif siloxy de formule $(CH_3)_3SiO_{1/2}$
    - $M^{Vi}$ est un motif siloxy de formule : $(CH_3)_2(vinyle)SiO_{1/2}$ ; et
    - Q est un motif siloxy de formule $SiO_{4/2}$,
- **EXT** = POS $\mathbf{B1}^{all}$: huile poly(diméthylsiloxy)-α, ω diméthylhydrogénosiloxy de viscosité d'environ 8,5 mPa.s et contenant en moyenne 5,7% en poids de motif SiH ; structure du type :
    - $M^HD_xM^H$ avec x compris en moyenne entre 7 et 15 ;
- **XL** = POS $\mathbf{B1}^{\mathbf{rét}}$: huile poly(diméthylsiloxy) (méthylhydrogénosiloxy) α, ω diméthylhydrogénosiloxy de viscosité moyenne de 70 mPa.s et contenant environ 1,9 % en poids de groupement SiH ; structure du type :
    - $M^H\ D_x\ D_w{}^H\ M^H$ avec x compris en moyenne entre 70 et 80 et w en moyenne entre 1 et 3 ;
- Cata. (C) = complexe organométallique du platine utilisé comme catalyseur de la réaction.
- ECH = inhibiteur de la réaction d'hydrosilylation = 1 -éthynyl-1 -cyclohexanol
- Stabilisant **K**= ester silylé de l'acide phosphorique.

b) Préparation des composites (=support enduit d'un gel silicone)

Les compositions silicones testées se présentent sous la forme bicomposante. Les parties nommées Partie A et Partie B sont mélangées ensuite dans un rapport en poids de 1 :1. Puis on applique la composition silicone précurseur d'un gel à un poids de 200 g/m² sur un support PET (épaisseur de 36 μm) à l'aide d'une racle d'enduction. Après l'enduction, on effectue la réticulation du composite pendant 30 min à 120°C en étuve ventilée de manière à obtenir un support enduit par un gel.

- Pour le composite n°1, on utilise un gel obtenu à partir de la composition silicone précurseur du gel et vendu sous l'appellation « Gel silicone Silbione® HC2 2022 » vendu par la société Bluestar Silicones.
- Pour le composite n°2, on utilise un gel obtenu à partir de la composition silicone suivante (mélange des parties A et B, ratio massique 1:1)

Tableau 1 : Composition du gel pour le composite n°2

| PARTIE A | | PARTIE B | |
|---|---|---|---|
| Constituants | % poids | Constituants | % poids |
| POS **A1** | 2,989 | POS **A1** | 26,65 |
| POS A**2** | 97,000 | POS **A2** | 55,90 |
| Cata. (C) | 0,066 | ECH | 0,012 |
| | | Résine silicone **Z¹** | 2,50 |
| | | POS **A3** | 7,50 |
| | | Stabilisant **K** | 0,04 |
| | | **XL** | 5,70 |
| | | **EXT** | 1,74 |

- Formulation n° 2 : $RH_{alk}$ = 0.62, poids en résine silicone **Z¹** = 1,25 % en poids par rapport au poids total de la composition et r1= 45%, viscosité après mélange des parties A et B =12000 mPa.s

- Pour le composite n°3, on utilise un gel obtenu à partir de la composition silicone suivante (mélange des parties A et B, ratio massique 1 : 1)

Tableau 2 : Composition du gel pour le composite n°3

| PARTIE A | | PARTIE B | |
|---|---|---|---|
| Constituants | % poids | Constituants | % poids |
| POS **A1** | 2,989 | POS **A1** | 21,96 |
| POS **A2** | 97,000 | POS **A2** | 49,41 |
| Cata. (C) | 0,066 | ECH | 0,012 |
| | | **Résine silicone $Z^1$** | 5,00 |
| | | POS **A3** | 15,00 |
| | | Stabilisant **K** | 0,04 |
| | | **XL** | 6,60 |
| | | **EXT** | 2,01 |

- Formulation n° 3 : $RH_{alk}$ = 0.62, poids en résine silicone **$Z^1$** = 2,50 % en poids par rapport au poids total de la composition et r1= 45%, viscosité après mélange des parties A et B = 10000 mPa.s

- Pour le composite n°4, on utilise un gel obtenu à partir de la composition silicone suivante (mélange des parties A et B, ratio massique 1 : 1)

Tableau 3 : Composition du gel pour le composite n°4

| PARTIE A | | PARTIE B | |
|---|---|---|---|
| Constituants | % poids | Constituants | % poids |
| POS **A1** | 2,989 | POS **A1** | 25,29 |
| POS **A2** | 97,000 | POS **A2** | 56,08 |
| Cata. (C) | 0,011 | ECH | 0,012 |
| | | **Résine silicone $Z^2$** | 4,00 |
| | | **POS A3** | 2,00 |
| | | POS **A1** | 4,00 |
| | | Stabilisant **K** | 0,04 |
| | | **XL** | 6,60 |
| | | **EXT** | 2,00 |

- Formulation n° 4: $RH_{alk}$ = 0.62, poids en résine **silicone $Z^2$** = 2 % en poids par rapport au poids total de la composition et r1= 45%, viscosité après mélange des parties A et B =15000 mPa.s.

- Pour le composite n°5 on utilise un gel obtenu à partir de la composition silicone suivante (mélange des parties A et B, ratio massique 1:1)

Tableau 4 : Composition du gel pour le composite n°5

| PARTIE A | | PARTIE B | |
|---|---|---|---|
| Constituants | % poids | Constituants | % poids |
| POS **A1** | 2,989 | POS **A1** | 21.29 |
| POS **A2** | 97,000 | POS **A2** | 47,77 |
| Cata. (C) | 0,011 | ECH | 0,012 |
| | | **Résine silicone $Z^2$** | 8,00 |
| | | POS **A3** | 4,00 |
| | | POS **A1** | 8,00 |
| | | Stabilisant **K** | 0,04 |
| | | **XL** | 8,38 |
| | | **EXT** | 2,55 |

- Formulation n° 5: $RH_{alk}$ = 0.62, poids en résine **silicone $Z^2$** = 4 % en poids par rapport au poids total de la composition et r1= 45%, viscosité après mélange des parties A et B =15000 mPa.s.

Tableau 5 : Propriétés des composites tests.

| Composites (supports enduit de gel silicone) | Résine silicone testée | Taux dans la composition silicone (%) | Pénétration (mm/10) | Peel 200g/m² (N/cm) | Tack (gf/cm²) | Shear (heure) |
|---|---|---|---|---|---|---|
| **n°1 Comparatif** | | 0 | 155 | 1.03 | 600 | <1 |
| **n°2 *Comparatif*** | résine, **silicone $Z^1$** | 1,25 | 154 | 1.19 | 758 | 3 |
| **n°3 *Invention*** | | 2,50 | 132 | 1.16 | 832 | 11 |
| ***n°4 Invention*** | résine **silicone $Z^2$** | 2,00 | 118 | 1.14 | 780 | 16 |
| ***n°5 comparatif*** | | 4,00 | Non mesuré | 0.3 | 292 | 13,7 |

L'introduction de la résine $Z^1$ à 2,50% en poids, par rapport au poids total de la composition, ou de la résine de résine $Z^2$ à 2% en poids, par rapport au poids total de la composition, permet d'atteindre des performances optimales (toutes les propriétés sont améliorées par rapport au comparatif composite n°1 et notamment la résistance au cisaillement tout en conservant des propriétés de tack optimales.

## Revendications

**1.** Article adhésif à la peau comprenant :

- un support **S** ayant une face de dessus **S1** et une face de dessous **S2,**
- éventuellement au moins un primaire d'accrochage **C1** appliqué sur au moins une partie ou sur la totalité de la face de dessus **S1** dudit support S,
- au moins une couche **D1** appliquée de manière continue ou discontinue sur la face de dessus **S1** dudit support **S** ou sur ledit primaire d'accrochage **C1** lorsqu'il est présent, et qui est constituée par un gel silicone **E** adhésif à la peau ayant les propriétés suivantes:

a) une pénétrabilité comprise entre 80 mm/10 et 300 mm/10, de préférence comprise entre 80 mm/10 et 200 mm/10, mesurée selon la norme NF ISO 2137 avec un pénétromètre ayant une tige et un cône et dont la somme des poids est égale à 62,5 g, et

b) un collant instantané ou «tack » compris entre 600 gf/cm² et 900 gf/cm², de préférence entre 700 gf/cm² et 850 gf/cm² pour une couche de 200 g/m² enduite sur un support en PET ayant une épaisseur de 36 µm

et mesuré selon la norme ASTM D2979,
c) un pouvoir adhésif compris entre 1,05 N/cm et 1,25 N/cm, de préférence compris entre 1,10 N/cm et 1,20 N/cm, pour une couche de 200 g/$m^2$ enduite sur un support en PET ayant une épaisseur de 36 $\mu$m et mesuré selon la méthode de test FINAT no. 1 par mise en contact sur une bande de papier bristol avec un angle de pelage de 180°, et
d) une résistance au cisaillement statique à 23°C ou « shear » supérieure à 3 heures mesurée selon la méthode de test FINAT no. 8 pour une couche de 200 g/$m^2$ enduite sur un support en PET ayant une épaisseur de 36 $\mu$m ; et

• éventuellement au moins une couche de protection **F** constituée d'un matériau de protection pelable et appliquée sur ladite couche **D1,**

le dit gel silicone **E** étant obtenu par réticulation d'une composition silicone **X** comprenant :

1) au moins un organopolysiloxane **A** comportant, par molécule, au moins deux radicaux alcényles en $C_2$ à $C_6$ liés chacun à un atome de silicium, et constitué :

(i) d'au moins deux motifs de formule (A1) :

$$(Y)a(Z)_bSiO_{(4-(a+b)/2} \qquad (A1)$$

dans laquelle :

- Y représente un groupe alcényle en $C_2$ à $C_6$, et de préférence un groupe vinyle,
- Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle;
- a et b représentent des nombres entiers, a valant 1, 2 ou 3, b valant 0, 1 ou 2 et (a+b) valant 2 ou 3;

(ii) et éventuellement d'au moins un motif de formule (A2) :

$$(Z)_cSiO_{(4-c)/2} \qquad (A2)$$

dans laquelle :

- Z a la même signification que ci-dessus, et
- c représente un nombre entier valant 2 ou 3.

2) au moins un organopolysiloxane **B** comportant, par molécule, au moins deux atomes d'hydrogène liés chacun à un atome de silicium, et de préférence au moins trois atomes d'hydrogène liés chacun à un atome de silicium,
3) au moins un catalyseur **C** d'hydrosilylation,
4) au moins un inhibiteur **D** de la réaction d'hydrosilylation,
5) éventuellement au moins un additif **K,** et
6) entre 1,5 % et 3,5 % en poids, et de préférence entre 1,75 % et 3,0 % en poids, par rapport au poids total de la composition silicone **X** d'au moins une résine silicone **Z** ayant des groupements alcényles liés à des atomes de silicium et comprenant :

a) au moins un motif siloxyle de formule **(I)** :

$$Y\,R_aSiO_{\frac{(3-a)}{2}}$$

**(I)**

dans laquelle :

- Y représente un groupe alcényle en $C_2$ à $C_6$. et de préférence un groupe vinyle,

- R est un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle, et
- a = 0, 1 ou 2, et de préférence a = 1 ou 2

b) au moins un motif siloxy de formule **(II)** :

$$R_bSiO_{\frac{(4-b)}{2}}$$

**(II)**

dans laquelle R ayant la même définition que ci-dessus et b = 1, 2 ou 3 ; et
c) au moins un motif siloxy Q de formule **(III)** :

$$SiO_{\frac{4}{2}}$$

**(III)**

avec les conditions suivantes :

a) les quantités en poids des organopolysiloxanes **A**, **B** et **Z** sont déterminées de manière à ce que la valeur du rapport $RH_aik = n_H/t_{Alk}$ soit comprise dans l'intervalle suivant : $0,10 \leq RH_{alk} \leq 0,80$, de préférence dans l'intervalle suivant $0,20 \leq RH_{alk} \leq 0,80$, et encore plus préférentiellement dans l'intervalle suivant $0,20 \leq RH_{alk} \leq 0,75$, avec $n_H$ = nombre de mole d'atome d'hydrogène directement lié à un atome de silicium de l'organopolysiloxane **B** et $t_{Alk}$ = nombre de mole d'alcényle directement lié à un atome de silicium de l'organopolysiloxane **A** et de la résine silicone **Z**, et
b) les viscosités et les quantités en poids des constituants de la composition silicone **X** sont choisies de manière à ce que la viscosité de la composition silicone **X** soit comprise entre 200 mPa.s et 100000 mPa.s à 25°C, et de préférence comprise entre 200 mPa.s et 80000 mPa.s à 25°C.

**2.** Article adhésif à la peau selon la revendication 1 comprenant :

• un support **S** ayant une face de dessus **S1** et une face de dessous **S2,**
• au moins un primaire d'accrochage **C1** appliqué sur au moins une partie ou sur la totalité de la face de dessus **S1** dudit support S,
• au moins une couche **D1** appliquée de manière continue ou discontinue sur ledit primaire d'accrochage **C1**, et qui est constituée par un gel silicone **E** adhésif à la peau ayant les propriétés suivantes :

a) une pénétrabilité comprise entre 80 mm/10 et 300 mm/10, de préférence comprise entre 80 mm/10 et 200 mm/10, mesurée selon la norme NF ISO 2137 avec un pénétromètre ayant une tige et un cône et dont la somme des poids est égale à 62,5 g, et
b) un collant instantané ou «tack » compris entre 600 $gf/cm^2$ et 900 $gf/cm^2$, de préférence entre 700 $gf/cm^2$ et 850 $gf/cm^2$ pour une couche de 200 $g/m^2$ enduite sur un support en PET ayant une épaisseur de 36 $\mu$m et mesuré selon la norme ASTM D2979,
c) un pouvoir adhésif compris entre 1,05 N/cm et 1,25 N/cm, de préférence compris entre 1,10N/cm et 1,20 N/cm, pour une couche de 200 $g/m^2$ enduite sur un support en PET ayant une épaisseur de 36 $\mu$m et mesuré selon la méthode de test FINAT no. 1 par mise en contact sur une bande de papier bristol avec un angle de pelage de 180°, et
d) une résistance au cisaillement statique à 23°C ou « shear » supérieure à 3 heures mesurée selon la méthode de test FINAT no. 8 pour une couche de 200 $g/m^2$ enduite sur un support en PET ayant une épaisseur de 36 $\mu$m ; et

• éventuellement au moins une couche de protection **F** constituée d'un matériau de protection pelable et appliquée sur ladite couche **D1,**

le dit gel silicone **E** étant obtenu par réticulation d'une composition silicone **X** comprenant :

1) au moins un organopolysiloxane **A** comportant, par molécule, au moins deux radicaux alcényles en $C_2$ à $C_6$ liés chacun à un atome de silicium, et constitué :

(i) d'au moins deux motifs siloxy de formule (A1) :

$$(Y)a(Z)_bSiO_{(4-(a+b)/2} \qquad (A1)$$

dans laquelle :

- Y représente un groupe alcényle en $C_2$ à $C_6$, et de préférence un groupe vinyle,
- Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle;
- a et b représentent des nombres entiers, a valant 1, 2 ou 3, b valant 0, 1 ou 2 et (a+b) valant 2 ou 3;

(ii) et éventuellement d'au moins un motif siloxy de formule (A2) :

$$(Z)_cSiO_{(4-c)/2} \qquad (A2)$$

dans laquelle :

- Z a la même signification que ci-dessus, et
- c représente un nombre entier valant 2 ou 3.

2) au moins un organopolysiloxane **B** comportant, par molécule, au moins deux atomes d'hydrogène liés chacun à un atome de silicium, et de préférence au moins trois atomes d'hydrogène liés chacun à un atome de silicium,
3) au moins un catalyseur **C** d'hydrosilylation,
4) au moins un inhibiteur **D** de la réaction d'hydrosilylation,
5) éventuellement au moins un additif **K,** et
6) entre 1,5 % et 3,5 % en poids, et de préférence entre 1,75 % et 3,0 % en poids, par rapport au poids total de la composition silicone **X** d'au moins une résine silicone **Z** ayant des groupements alcényles liés à des atomes de silicium et comprenant :

a) au moins un motif siloxy de formule **(I)** :

$$Y R_a SiO_{\frac{(3-a)}{2}} \qquad \textbf{(I)}$$

dans laquelle :

- Y représente un groupe alcényle en $C_2$ à $C_6$, et de préférence un groupe vinyle,
- R est un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle, et
- a = 0, 1 ou 2, et de préférence a = 1 ou 2

b) au moins un motif siloxy de formule **(II)** :

$$R_b SiO_{\frac{(4-b)}{2}} \qquad \textbf{(II)}$$

dans laquelle R ayant la même définition que ci-dessus et b = 1, 2 ou 3 ; et
c) au moins un motif siloxy Q de formule **(III)** :

$$SiO_{\frac{4}{2}}$$

**(III)**

avec les conditions suivantes :

a) les quantités en poids des organopolysiloxanes **A, B** et **Z** sont déterminées de manière à ce que la valeur du rapport $RH_alk = n_H/t_{Alk}$ soit comprise dans l'intervalle suivant : 0,10 s $RH_{alk} \leq 0,80$, de préférence dans l'intervalle suivant $0,20 \leq RH_{alk} \leq 0,80$, et encore plus préférentiellement dans l'intervalle suivant $0,20 \leq RH_{alk} \leq 0,75$, avec $n_H$ = nombre de mole d'atome d'hydrogène directement lié à un atome de silicium de l'organopolysiloxane **B** et $t_{Alk}$ = nombre de mole d'alcényle directement lié à un atome de silicium de l'organopolysiloxane **A** et de la résine silicone **Z**, et
b) les viscosités et les quantités en poids des constituants de la composition silicone **X** sont choisies de manière à ce que la viscosité dynamique à 25°C de la composition silicone **X** soit comprise entre 200 mPa.s et 100000 mPa.s, et de préférence comprise entre 200 mPa.s et 80000 mPa.s.

**3.** Article adhésif à la peau selon la revendication 1 **caractérisé en ce que** la résine silicone **Z** ayant des groupements alcényles liés à des atomes de silicium est choisie parmi le groupe constitué par :

- une résine silicone $\mathbf{Z^1}$ de formule $MD^{vi}Q$ dans laquelle :

• M est un motif siloxy de formule $R_3SiO_{1/2}$ dans laquelle R est un alkyle en $C_1$ à $C_8$ ou un groupe aryle tel que xylyle, tolyle ou phényle,
• $D^{Vi}$ est un motif siloxy de formule $RR^1SiO_{2/2}$ avec R étant un groupe alkyle en $C_1$ à $C_8$ ou un groupe aryle et $R^1$ étant un groupe vinyle, et
• Q est un motif siloxy de formule $SiO_{4/2}$ ;

- une résine silicone $\mathbf{Z^2}$ de formule $MM^{Vi}Q$ dans laquelle :

• M est un motif siloxy de formule $R_3SiO_{1/2}$ dans laquelle R est un alkyle en $C_1$ à $C_8$ ou un groupe aryle tel que xylyle, tolyle ou phényle,
• $M^{Vi}$ est un motif siloxy de formule $R_2(Vi)SiO_{1/2}$ avec Vi étant un groupe vinyle et R étant un groupe alkyle en $C_1$ à $C_8$ ou un groupe aryle tel que xylyle, tolyle ou phényle, et
• Q est un motif siloxy de formule $SiO_{4/2}$, et

- les mélanges des résines silicones $\mathbf{Z^1}$ et $\mathbf{Z^2}$.

**4.** Article adhésif à la peau selon la revendication 1 ou 2 **caractérisé en ce que** la composition silicone **X** comprend au moins deux organopolysiloxanes **B** différents qui sont :

a) un allongeur de chaine $\mathbf{B^{all}}$ comprenant :

• des motifs siloxy monovalent terminaux, identiques ou différents, de formule (M) :

$$(H)_p(R^1)_qSiO_{1/2} \qquad (M)$$

dans laquelle le symbole $R^1$ correspond à un groupe alkyle en $C_1$ à $C_8$; le symbole H représente un atome d'hydrogène et avec p= 0 ou 1, q=2 ou 3 et (p+q)= 3;
• des motifs siloxy bivalent, identiques ou différents, de formule (D) :

$$(H)_n(R^2)_mSiO_{2/2} \qquad (D)$$

dans laquelle le radical $R^2$ correspond à un groupe alkyle en $C_1$ à $C_8$ ou un groupe aryle, le symbole H

représente un atome d'hydrogène et avec n= 0 ou 1, m =1 ou 2 et (n+m)= 2, et

• avec la condition selon laquelle l'organopolysiloxanes **$B^{all}$** comprend deux atomes d'hydrogène liés chacun à un atome de silicium différent par polymère, c'est-à-dire deux fonctions Si-H par polymère ;

b) un réticulant **$B^{ret}$** comprenant :

• au moins trois motifs siloxy de formule (B.1) :

$$(H)\ (L)_e\ SiO_{(3-e)/2} \qquad (B.1)$$

dans laquelle le symbole H représente un atome d'hydrogène, le symbole L représente un alkyle ayant de 1 à 8 atomes de carbone inclus ou un aryle tel que xylyle, tolyle ou phényle, et le symbole e est égal à 0, 1 ou 2 ; et

• éventuellement des autres motifs siloxy de formule (B-2) :

(L)g SiO(4-g)/2

dans laquelle le symbole L représente un alkyle ayant de 1 à 8 atomes de carbone inclus et le symbole g est égal à 0, 1, 2 ou 3.

**5.** Article adhésif à la peau selon la revendication 1 ou 2 dans lequel la composition silicone **X** comprend au moins deux organopolysiloxanes **A1** et **A2** comportant, par molécule, au moins deux radicaux alcényles en $C_2$ à $C_6$ liés chacun à un atome de silicium caractérisé en ce :

a) l'organopolysiloxanes **A1** à une viscosité dynamique à 25°C comprise entre 100 mPa.s et 120000 mPa.s, et
b) l'organopolysiloxanes **A2** est une gomme ayant une consistance à 25°C comprise entre 500 mm/10 et 1000 mm/10.

**6.** Article adhésif à la peau selon la revendication 1 ou 2 **caractérisé en ce que** la composition silicone **X** comprend au moins trois organopolysiloxanes **A** comportant, par molécule, au moins deux radicaux alcényles en $C_2$ à $C_6$ liés chacun à un atome de silicium :

a) le premier ayant une viscosité comprise entre 100 mPa.s et 5000 mPa.s,
b) le deuxième ayant une viscosité comprise entre 5000 mPa.s et 15000 mPa.s, et
c) le troisième ayant une viscosité comprise entre 15000 mPa.s et 100000 mPa.s.

**7.** Article adhésif à la peau selon la revendication 1 ou 2 **caractérisé en ce que** le support **S** est composé d'un matériau non-tissé, d'un matériau textile tricoté ou tissé, ou d'un film de matière plastique.

**8.** Article adhésif à la peau selon la revendication 7 **caractérisé en ce que** le support **S** est un film souple perforé en polyuréthane ou un film souple continu en polyuréthane.

**9.** Article adhésif à la peau selon la revendication 1 ou 8 **caractérisé en ce qu'**il comprend une ou plusieurs couches **N** comprenant un corps absorbant **O,** éventuellement séparées par une ou plusieurs couches intermédiaires **P,** placée(s) sur le support **S** du côté de la face de dessous **S2** du support **S.**

**10.** Article adhésif à la peau selon l'une quelconque des revendications 1, 2 et 8 **caractérisé en ce que** le support **S** est un film souple en polyuréthane et comporte sur au moins une partie de la face de dessous **S2** un adhésif sensible à la pression.

**11.** Article adhésif à la peau selon l'une quelconque des revendications précédentes **caractérisé en ce qu'**il s'agit d'un dispositif médical ou d'un élément d'un dispositif médical.

**Patentansprüche**

**1.** Artikel, der auf der Haut haftet, umfassend:

• einen Träger S mit einer Oberseite S1 und einer Unterseite S2,
• gegebenenfalls mindestens eine Haftgrundierung C1, die auf mindestens einen Teil oder die Gesamtheit der Oberseite S1 des Trägers S aufgebracht ist,
• mindestens eine Schicht D1, die kontinuierlich oder diskontinuierlich auf die Oberseite S1 des Trägers S oder die Haftgrundierung C1, wenn sie vorhanden ist, aufgebracht ist und die aus einem auf der Haut haftenden Silikongel E mit den folgenden Eigenschaften besteht:

a) einer gemäß der NF-ISO-Norm 2137 mit einem Penetrometer mit einem Stab und einem Kegel, deren Gesamtgewicht 62,5 g beträgt, gemessenen Penetration zwischen 80 mm/10 und 300 mm/10, vorzugsweise zwischen 80 mm/10 und 200 mm/10, und
b) einer gemäß der ASTM-Norm D2979 gemessenen Sofortklebrigkeit oder "Tack" zwischen 600 gf/$cm^2$ und 900 gf/$cm^2$, vorzugsweise zwischen 700 gf/$cm^2$ und 850 gf/$cm^2$, für eine auf einen PET-Träger mit einer Dicke von 36 $\mu$m gebrachte Schicht von 200 g/$m^2$,
c) einer gemäß der FINAT-Testmethode Nr. 1 durch Inkontaktbringen eines Streifens aus Bristol-Papier mit einem Abziehewinkel von 180° gemessenen Klebkraft zwischen 1,05 N/cm und 1,25 N/cm, vorzugsweise zwischen 1,10 N/cm und 1,20 N/cm, für eine auf einen PET-Träger mit einer Dicke von 36 $\mu$m gebrachte Schicht von 200 g/$m^2$ und
d) einem gemäß der FINAT-Testmethode Nr. 8 für eine auf einen PET-Träger mit einer Dicke von 36 $\mu$m gebrachte Schicht von 200 g/$m^2$ gemessenen Widerstand gegen statische Scherung bei 23 °C oder "Shear" von mehr als 3 Stunden; und

• gegebenenfalls mindestens eine Schutzschicht F, die aus einem abziehbaren Schutzmaterial besteht und auf die Schicht D1 aufgebracht ist,

wobei das Silikongel E durch Vernetzung einer Silikonzusammensetzung X erhalten wird, die Folgendes umfasst:

1) mindestens ein Organopolysiloxan A, das pro Molekül mindestens zwei jeweils an ein Siliciumatom gebundene $C_2$- bis $C_6$-Alkenylreste umfasst und aus Folgendem besteht:

(i) mindestens zwei Einheiten der Formel (A1):

$$(Y)a(Z)_bSiO_{(4-(a+b)/2} \qquad (A1)$$

in der:

- Y für eine $C_2$- bis $C_6$-Alkenylgruppe und vorzugsweise eine Vinylgruppe steht,
- Z für eine einwertige Kohlenwasserstoffgruppe steht, die aus Alkylgruppen mit 1 bis 8 Kohlenstoffatomen inklusive wie Methyl-, Ethyl-, Propyl- und 3,3,3-Trifluorpropylgruppen, Cycloalkylgruppen wie Cyclohexyl-, Cycloheptyl- und Cyclooctylgruppen und Arylgruppen wie Xylyl, Tolyl und Phenyl ausgewählt ist,
- a und b für ganze Zahlen stehen, wobei a einen Wert von 1, 2 oder 3 hat, b einen Wert von 0, 1 oder 2 hat und (a+b) einen Wert von 2 oder 3 hat;

(ii) und gegebenenfalls mindestens eine Einheit der Formel (A2):

$$(Z)_cSiO_{(4-c)/2} \qquad (A2)$$

in der:

- Z die gleiche Bedeutung wie oben hat und
- c für eine ganze Zahl mit einem Wert von 2 oder 3 steht;

2) mindestens ein Organopolysiloxan B, das pro Molekül mindestens zwei jeweils an ein Siliciumatom gebundene Wasserstoffatome und vorzugsweise mindestens drei jeweils an ein Siliciumatom gebundene Wasserstoffatome umfasst,
3) mindestens einen Hydrosilylierungskatalysator C,
4) mindestens einen Inhibitor D der Hydrosilylierungsreaktion,
5) gegebenenfalls mindestens ein Additiv K und

6) zwischen 1,5 und 3,5 Gew.-% vorzugsweise zwischen 1,75 und 3,0 Gew.-%, bezogen auf das Gesamtgewicht der Silikonzusammensetzung X, mindestens eines Silikonharzes Z, das an Siliciumatome gebundene Alkenylgruppen aufweist und Folgendes umfasst:

a) mindestens eine Siloxyleinheit der Formel (I) :

$$Y\,R_aSiO_{\frac{(3-a)}{2}} \quad (I)$$

in der:

- Y für eine $C_2$- bis $C_6$-Alkenylgruppe und vorzugsweise eine Vinylgruppe steht,
- R für eine einwertige Kohlenwasserstoffgruppe steht, die aus Alkylgruppen mit 1 bis 8 Kohlenstoffatomen inklusive wie Methyl-, Ethyl-, Propyl- und 3,3,3-Trifluorpropylgruppen, Cycloalkylgruppen wie Cyclohexyl-, Cycloheptyl- und Cyclooctylgruppen und Arylgruppen wie Xylyl, Tolyl und Phenyl ausgewählt ist, und
- a = 0, 1 oder 2 und vorzugsweise a = 1 oder 2;

b) mindestens eine Siloxyeinheit der Formel (II) :

$$R_bSiO_{\frac{(4-b)}{2}} \quad (II)$$

in der R die gleiche Definition wie oben hat und b = 1, 2 oder 3; und
c) mindestens eine Siloxyeinheit Q der Formel (III) :

$$SiO_{\frac{4}{2}} \quad (III)$$

mit den folgenden Bedingungen:

a) die Gewichtsmengen der Organopolysiloxane A, B und Z sind so bestimmt, dass der Wert des Verhältnisses $RH_{alk} = n_H/t_{Alk}$ im folgenden Intervall liegt: $0{,}10 \leq RH_{alk} \leq 0{,}80$, vorzugsweise im folgenden Intervall $0{,}20 \leq RH_{alk} \leq 0{,}80$ und noch weiter bevorzugt im folgenden Intervall $0{,}20 \leq RH_{alk} \leq 0{,}75$ liegt, wobei $n_H$ = Zahl der Mole von direkt an ein Siliciumatom des Organopolysiloxans B gebundenen Wasserstoffatomen und $t_{Alk}$ = Zahl der Mole von direkt an ein Siliciumatom des Organopolysiloxans A und des Silikonharzes Z gebundenen Alkenylgruppen, und
b) die Viskositäten und die Gewichtsmengen der Bestandteile der Silikonzusammensetzung X sind so gewählt, dass die Viskosität der Silikonzusammensetzung X zwischen 200 mPa.s und 100.000 mPa.s bei 25 °C und vorzugsweise zwischen 200 mPa.s und 80.000 mPa.s bei 25 °C liegt.

**2.** Artikel, der auf der Haut haftet, nach Anspruch 1, umfassend:

• einen Träger S mit einer Oberseite S1 und einer Unterseite S2,
• mindestens eine Haftgrundierung C1, die auf mindestens einen Teil oder die Gesamtheit der Oberseite S1 des Trägers S aufgebracht ist,
• mindestens eine Schicht D1, die kontinuierlich oder diskontinuierlich auf die Haftgrundierung C1 aufgebracht ist und die aus einem auf der Haut haftenden Silikongel E mit den folgenden Eigenschaften besteht:

a) einer gemäß der NF-ISO-Norm 2137 mit einem Penetrometer mit einem Stab und einem Kegel, deren Gesamtgewicht 62,5 g beträgt, gemessenen Penetration zwischen 80 mm/10 und 300 mm/10, vorzugsweise zwischen 80 mm/10 und 200 mm/10, und
b) einer gemäß der ASTM-Norm D2979 gemessenen Sofortklebrigkeit oder "Tack" zwischen 600 gf/cm$^2$ und 900 gf/cm$^2$, vorzugsweise zwischen 700 gf/cm$^2$ und 850 gf/cm$^2$, für eine auf einen PET-Träger mit

einer Dicke von 36 μm gebrachte Schicht von 200 $g/m^2$,
c) einer gemäß der FINAT-Testmethode Nr. 1 durch Inkontaktbringen eines Streifens aus Bristol-Papier mit einem Abziehwinkel von 180° gemessenen Klebkraft zwischen 1,05 N/cm und 1,25 N/cm, vorzugsweise zwischen 1,10 N/cm und 1,20 N/cm, für eine auf einen PET-Träger mit einer Dicke von 36 μm gebrachte Schicht von 200 $g/m^2$ und
d) einem gemäß der FINAT-Testmethode Nr. 8 für eine auf einen PET-Träger mit einer Dicke von 36 μm gebrachte Schicht von 200 $g/m^2$ gemessenen Widerstand gegen statische Scherung bei 23 °C oder "Shear" von mehr als 3 Stunden; und

• gegebenenfalls mindestens eine Schutzschicht F, die aus einem abziehbaren Schutzmaterial besteht und auf die Schicht D1 aufgebracht ist,

wobei das Silikongel E durch Vernetzung einer Silikonzusammensetzung X erhalten wird, die Folgendes umfasst:

1) mindestens ein Organopolysiloxan A, das pro Molekül mindestens zwei jeweils an ein Siliciumatom gebundene $C_2$- bis $C_6$-Alkenylreste umfasst und aus Folgendem besteht:

(i) mindestens zwei Einheiten der Formel (A1):

$$(Y)a(Z)_bSiO_{(4-(a+b)/2} \qquad (A1)$$

in der:

- Y für eine $C_2$- bis $C_6$-Alkenylgruppe und vorzugsweise eine Vinylgruppe steht,
- Z für eine einwertige Kohlenwasserstoffgruppe steht, die aus Alkylgruppen mit 1 bis 8 Kohlenstoffatomen inklusive wie Methyl-, Ethyl-, Propyl- und 3,3,3-Trifluorpropylgruppen, Cycloalkylgruppen wie Cyclohexyl-, Cycloheptyl- und Cyclooctylgruppen und Arylgruppen wie Xylyl, Tolyl und Phenyl ausgewählt ist,
- a und b für ganze Zahlen stehen, wobei a einen Wert von 1, 2 oder 3 hat, b einen Wert von 0, 1 oder 2 hat und (a+b) einen Wert von 2 oder 3 hat;

(ii) und gegebenenfalls mindestens eine Einheit der Formel (A2):

$$(Z)_cSiO_{(4-c)/2} \qquad (A2)$$

in der:

- Z die gleiche Bedeutung wie oben hat und
- c für eine ganze Zahl mit einem Wert von 2 oder 3 steht;

2) mindestens ein Organopolysiloxan B, das pro Molekül mindestens zwei jeweils an ein Siliciumatom gebundene Wasserstoffatome und vorzugsweise mindestens drei jeweils an ein Siliciumatom gebundene Wasserstoffatome umfasst,
3) mindestens einen Hydrosilylierungskatalysator C,
4) mindestens einen Inhibitor D der Hydrosilylierungsreaktion,
5) gegebenenfalls mindestens ein Additiv K und
6) zwischen 1,5 und 3,5 Gew.-% vorzugsweise zwischen 1,75 und 3,0 Gew.-%, bezogen auf das Gesamtgewicht der Silikonzusammensetzung X, mindestens eines Silikonharzes Z, das an Siliciumatome gebundene Alkenylgruppen aufweist und Folgendes umfasst:

a) mindestens eine Siloxyleinheit der Formel (I) :

$$Y\,R_aSiO_{\frac{(3-a)}{2}} \qquad (I)$$

in der:

- Y für eine $C_2$- bis $C_6$-Alkenylgruppe und vorzugsweise eine Vinylgruppe steht,
- R für eine einwertige Kohlenwasserstoffgruppe steht, die aus Alkylgruppen mit 1 bis 8 Kohlenstoffatomen inklusive wie Methyl-, Ethyl-, Propyl- und 3,3,3-Trifluorpropylgruppen, Cycloalkylgruppen wie Cyclohexyl-, Cycloheptyl- und Cyclooctylgruppen und Arylgruppen wie Xylyl, Tolyl und Phenyl ausgewählt ist, und
- a = 0, 1 oder 2 und vorzugsweise a = 1 oder 2;

b) mindestens eine Siloxyeinheit der Formel (II) :

$$R_bSiO_{\frac{(4-b)}{2}} \qquad (II)$$

in der R die gleiche Definition wie oben hat und b = 1, 2 oder 3; und
c) mindestens eine Siloxyeinheit Q der Formel (III) :

$$SiO_{\frac{4}{2}} \qquad (III)$$

mit den folgenden Bedingungen:

a) die Gewichtsmengen der Organopolysiloxane A, B und Z sind so bestimmt, dass der Wert des Verhältnisses $RH_{alk} = n_H/t_{Alk}$ im folgenden Intervall liegt: $0{,}10 \leq RH_{alk} \leq 0{,}80$, vorzugsweise im folgenden Intervall $0{,}20 \leq RH_{alk} \leq 0{,}80$ und noch weiter bevorzugt im folgenden Intervall $0{,}20 \leq RH_{alk} \leq 0{,}75$ liegt, wobei $n_H$ = Zahl der Mole von direkt an ein Siliciumatom des Organopolysiloxans B gebundenen Wasserstoffatomen und $t_{Alk}$ = Zahl der Mole von direkt an ein Siliciumatom des Organopolysiloxans A und des Silikonharzes Z gebundenen Alkenylgruppen, und
b) die Viskositäten und die Gewichtsmengen der Bestandteile der Silikonzusammensetzung X sind so gewählt, dass die Viskosität der Silikonzusammensetzung X zwischen 200 mPa.s und 100.000 mPa.s bei 25 °C und vorzugsweise zwischen 200 mPa.s und 80.000 mPa.s bei 25 °C liegt.

**3.** Artikel, der auf der Haut haftet, nach Anspruch 1, **dadurch gekennzeichnet, dass** das Silikonharz Z, das an Siliciumatome gebundene Alkenylgruppen aufweist, aus der Gruppe bestehend aus

- einem Silikonharz $Z^1$ der Formel $MD^{Vi}Q$, in der:

• M für eine Siloxyeinheit der Formel $R_3SiO_{1/2}$ steht, in der R für ein $C_1$- bis $C_8$-Alkyl oder eine Arylgruppe wie Xylyl, Tolyl oder Phenyl steht,
• $D^{Vi}$ für eine Siloxyeinheit der Formel $RR^1SiO_{2/2}$ steht, wobei R für ein $C_1$- bis $C_8$-Alkyl oder eine Arylgruppe steht und $R^1$ für eine Vinylgruppe steht, und
• Q für eine Siloxyeinheit der Formel $SiO_{4/2}$ steht;

- einem Silikonharz $Z^2$ der Formel $MM^{Vi}Q$, in der:

• M für eine Siloxyeinheit der Formel $R_3SiO_{1/2}$ steht, in der R für ein $C_1$- bis $C_8$-Alkyl oder eine Arylgruppe wie Xylyl, Tolyl oder Phenyl steht,
• $M^{Vi}$ für eine Siloxyeinheit der Formel $R_2(Vi)SiO_{1/2}$ steht, wobei Vi für eine Vinylgruppe steht und R für ein $C_1$- bis $C_8$-Alkyl oder eine Arylgruppe wie Xylyl, Tolyl oder Phenyl steht, und
• Q für eine Siloxyeinheit der Formel $SiO_{4/2}$ steht; und

- Mischungen der Silikonharze $Z^1$ und $Z^2$ ausgewählt ist.

**4.** Artikel, der auf der Haut haftet, nach Anspruch 1 oder 2, **dadurch gekennzeichnet, dass** die Silikonzusammensetzung X mindestens zwei verschiedene Organosiloxane B umfasst, bei denen es sich um Folgendes handelt:

a) ein Kettenverlängerungsmittel $B^{all}$, umfassend:

• gleiche oder verschiedene endständige einwertige Silyloxyeinheiten der Formel (M):

$$(H)_p(R^1)_qSiO_{1/2} \qquad (M)$$

in der das Symbol $R^1$ einer $C_1$- bis $C_8$-Alkylgruppe entspricht, das Symbol H für ein Wasserstoffatom steht und wobei p = 0 oder 1, q = 2 oder 3 und (p+q) = 3;
• gleiche oder verschiedene zweiwertige Siloxyeinheiten der Formel (D):

$$(H)_n(R^2)_mSiO_{2/2} \qquad (D)$$

in der das Symbol $R^2$ einer $C_1$- bis $C_8$-Alkylgruppe oder einer Arylgruppe entspricht, das Symbol H für ein Wasserstoffatom steht und wobei n = 0 oder 1, m = 1 oder 2 und (n+m) = 2, und
• mit der Bedingung, gemäß der das Organopolysiloxan $B^{all}$ zwei jeweils an ein unterschiedliches Siliciumatom gebundene Wasserstoffatome pro Polymer, d. h. zwei Si-H-Funktionen pro Polymer, umfasst;

b) ein Vernetzungsmittel $B^{ret}$, umfassend:

• mindestens drei Siloxyeinheiten der Formel (B.1) :

$$(H)\ (L)\ eSiO_{(3-e)/2} \qquad (B.1)$$

in der das Symbol H für ein Wasserstoffatom steht, das Symbol L für ein Alkyl mit 1 bis 8 Kohlenstoffatomen inklusive oder ein Aryl wie Xylyl, Tolyl oder Phenyl steht und das Symbol e gleich 0, 1 oder 2 ist; und
• gegebenenfalls andere Siloxyeinheiten der Formel (B-2):

$$(L)_gSiO_{(4-g)/2}$$

in der das Symbol L für ein Alkyl mit 1 bis 8 Kohlenstoffatomen inklusive steht und das Symbol g gleich 0, 1, 2 oder 3 ist.

**5.** Artikel, der auf der Haut haftet, nach Anspruch 1 oder 2, wobei die Silikonzusammensetzung X mindestens zwei Organopolysiloxane A1 und A2 umfasst, die pro Molekül mindestens zwei jeweils an ein Siliciumatom gebundene $C_2$- bis $C_6$-Alkenylreste umfassen, **dadurch gekennzeichnet, dass**:

a) das Organopolysiloxan A1 eine dynamische Viskosität bei 25 °C zwischen 100 mPa.s und 120.000 mPa.s aufweist und
b) es sich bei dem Organosiloxane A2 um ein Gummi mit einer Konsistenz bei 25 °C zwischen 500 mm/10 und 1000 mm/10 handelt.

**6.** Artikel, der auf der Haut haftet, nach Anspruch 1 oder 2, **dadurch gekennzeichnet, dass** die Silikonzusammensetzung X mindestens drei Organopolysiloxane umfasst, die pro Molekül mindestens zwei jeweils an ein Siliciumatom gebundene $C_2$- bis $C_6$-Alkenylreste umfassen, wobei:

a) das erste eine Viskosität zwischen 100 mPa.s und 5000 mPa.s aufweist,
b) das zweite eine Viskosität zwischen 5000 mPa.s und 15.000 mPa.s aufweist und
c) das dritte eine Viskosität zwischen 15.000 mPa.s und 100.000 mPa.s aufweist.

**7.** Artikel, der auf der Haut haftet, nach Anspruch 1 oder 2, **dadurch gekennzeichnet, dass** der Träger S aus einem Vliesstoff, einer textilen Maschen- oder Webware oder einer Kunststofffolie besteht.

**8.** Artikel, der auf der Haut haftet, nach Anspruch 7, **dadurch gekennzeichnet, dass** es sich bei dem Träger S um eine perforierte flexible Polyurethanfolie oder eine kontinuierliche weiche Polyurethanfolie handelt.

**9.** Artikel, der auf der Haut haftet, nach Anspruch 1 oder 8, **dadurch gekennzeichnet, dass** er eine oder mehrere Schichten N, die eine absorbierende Substanz umfassen, umfasst, die gegebenenfalls durch eine oder mehrere Zwischenschichten P, die auf dem Träger S auf der Unterseite S2 des Trägers S angeordnet sind, getrennt sind.

**10.** Artikel, der auf der Haut haftet, nach einem der Ansprüche 1, 2 und 8, **dadurch gekennzeichnet, dass** der Träger

S eine flexible Polyurethanfolie ist und auf mindestens einem Teil der Unterseite S2 einen Haftklebstoff umfasst.

**11.** Artikel, der auf der Haut haftet, nach einem der vorhergehenden Ansprüche, **dadurch gekennzeichnet, dass** es sich um eine medizinische Vorrichtung oder ein Element einer medizinischen Vorrichtung handelt.

## Claims

**1.** Item that adheres to the skin, comprising:

- a support S having a top face S1 and a bottom face S2,
- optionally at least one tie primer C1 applied on at least one part or on all of the top face S1 of said support S,
- at least one layer D1 applied continuously or discontinuously on the top face S1 of said support S or on said tie primer C1 when it is present, and which consists of a silicone gel E that adheres to the skin, having the following properties:

  a) a penetrability of between 80 mm/10 and 300 mm/10, preferably of between 80 mm/10 and 200 mm/10, measured according to the standard NF ISO 2137 with a penetrometer having a rod and a cone and the sum of the weights of which is equal to 62.5 g, and
  b) a tack of between 600 gf/cm$^2$ and 900 gf/cm$^2$, preferably between 700 gf/cm$^2$ and 850 gf/cm$^2$ for a layer of 200 g/m$^2$ coated onto a PET support having a thickness of 36 $\mu$m and measured according to the standard ASTM D2979,
  c) an adhesive power of between 1.05 N/cm and 1.25 N/cm, preferably of between 1.10 N/cm and 1.20 N/cm, for a layer of 200 g/m$^2$ coated onto a PET support having a thickness of 36 $\mu$m and measured according to the FINAT no. 1 test method by bringing into contact with a strip of Bristol paper with a peel angle of 180°, and
  d) a static shear strength at 23°C greater than 3 hours measured according to the FINAT no. 8 test method for a layer of 200 g/m$^2$ coated onto a PET support having a thickness of 36 $\mu$m; and

- optionally at least one protective layer F consisting of a peel-off protective material and applied on said layer D1,

said silicone gel E being obtained by crosslinking of a silicone composition X comprising:

1) at least one organopolysiloxane A comprising, per molecule, at least two $C_2$-$C_6$ alkenyl radicals each bonded to a silicon atom and consisting:

(i) of at least two units of formula (A1):

$$(Y)a\ (Z)_bSiO_{(4-(a+b)/2} \qquad (A1)$$

in which:

- Y represents a $C_2$ to $C_6$ alkenyl group, and preferably a vinyl group,
- Z represents a monovalent hydrocarbon-based group chosen from alkyl groups having from 1 to 8 carbon atoms inclusive, such as methyl, ethyl, propyl and 3,3,3-trifluoropropyl groups, cycloalkyl groups, such as cyclohexyl, cycloheptyl or cyclooctyl groups, and aryl groups such as xylyl, tolyl and phenyl groups;
- a and b represent integers, a being 1, 2 or 3, b being 0, 1 or 2 and (a+b) being 2 or 3;

(ii) and optionally of at least one unit of formula (A2) :

$$(Z)_cSiO_{(4-c/2} \qquad (A2)$$

in which:

- Z has the same meaning as above, and
- c represents an integer which is 2 or 3,

2) at least one organopolysiloxane B comprising, per molecule, at least two hydrogen atoms each bonded to a silicon atom, and preferably at least three hydrogen atoms each bonded to a silicon atom,
3) at least one hydrosilylation catalyst C,
4) at least one hydrosilylation reaction inhibitor D,
5) optionally at least one additive K, and
6) between 1.5% and 3.5% by weight, and preferably between 1.75% and 3.0% by weight, relative to the total weight of the silicone composition X , of at least one silicone resin Z having alkenyl groups bonded to silicon atoms and comprising:

a) at least one siloxyl unit of formula (I):

$$Y\,R_aSiO_{\frac{(3-a)}{2}} \quad (I)$$

in which:

- Y represents a $C_2$ to $C_6$ alkenyl group, and preferably a vinyl group,
- R is a monovalent hydrocarbon-based group chosen from alkyl groups having from 1 to 8 carbon atoms inclusive, such as methyl, ethyl, propyl and 3,3,3-trifluoropropyl groups, cycloalkyl groups, such as cyclohexyl, cycloheptyl or cyclooctyl groups, and aryl groups such as xylyl, tolyl and phenyl groups, and
- a = 0, 1 or 2, and preferably a = 1 or 2

b) at least one siloxy unit of formula (II):

$$R_bSiO_{\frac{(4-b)}{2}} \quad (II)$$

in which R has the same definition as above and b = 1, 2 or 3; and
c) at least one siloxy unit Q of formula (III):

$$SiO_{\frac{4}{2}} \quad (III)$$

with the following conditions:

a) the amounts by weight of the organopolysiloxanes A, B and Z are determined such that the value of the ratio $RH_{alk} = n_H/t_{Alk}$ is included in the following range: $0.10 \leq RH_{alk} \leq 0.80$, preferably in the following range $0.20 \leq RH_{alk} \leq 0.80$, and even more preferentially in the following range $0.20 \leq RH_{alk} \leq 0.75$, with $n_H$ = number of moles of hydrogen atom directly bonded to a silicon atom of the organopolysiloxane B and $t_{Alk}$ = number of moles of alkenyl directly bonded to a silicon atom of the organopolysiloxane A and of the silicone resin Z, and
b) the viscosities and the amounts by weight of the constituents of the silicone composition X are chosen such that the viscosity of the silicone composition X is between 200 mPa.s and 100 000 mPa.s at 25°C, and preferably between 200 mPa.s and 80 000 mPa.s at 25°C.

**2.** Item that adheres to the skin according to Claim 1, comprising:

• a support S having a top face S1 and a bottom face S2,
• at least one tie primer C1 applied on at least one part or on all of the top face S1 of said support S,
• at least one layer D1 applied continuously or discontinuously on said tie primer C1, and which consists of a silicone gel E that adheres to the skin, having the following properties:

a) a penetrability of between 80 mm/10 and 300 mm/10, preferably of between 80 mm/10 and 200 mm/10, measured according to the standard NF ISO 2137 with a penetrometer having a rod and a cone and the sum of the weights of which is equal to 62.5 g, and

b) a tack of between 600 gf/$cm^2$ and 900 gf/$cm^2$, preferably between 700 gf/$cm^2$ and 850 gf/$cm^2$ for a layer of 200 g/$m^2$ coated onto a PET support having a thickness of 36 μm and measured according to the standard ASTM D2979,

c) an adhesive power of between 1.05 N/cm and 1.25 N/cm, preferably of between 1.10 N/cm and 1.20 N/cm, for a layer of 200 g/$m^2$ coated onto a PET support having a thickness of 36 μm and measured according to the FINAT no. 1 test method by bringing into contact with a strip of Bristol paper with a peel angle of 180°, and

d) a static shear strength at 23°C greater than 3 hours measured according to the FINAT no. 8 test method for a layer of 200 g/$m^2$ coated onto a PET support having a thickness of 36 μm; and

• optionally at least one protective layer F consisting of a peel-off protective material and applied on said layer D1,

said silicone gel E being obtained by crosslinking of a silicone composition X comprising:

1) at least one organopolysiloxane A comprising, per molecule, at least two $C_2$-$C_6$ alkenyl radicals each bonded to a silicon atom and consisting:

(i) of at least two siloxy units of formula (A1):

$$(Y)a(Z)_bSiO_{(4-(a+b)/2} \qquad (A1)$$

in which:

- Y represents a $C_2$ to $C_6$ alkenyl group, and preferably a vinyl group,
- Z represents a monovalent hydrocarbon-based group chosen from alkyl groups having from 1 to 8 carbon atoms inclusive, such as methyl, ethyl, propyl and 3,3,3-trifluoropropyl groups, cycloalkyl groups, such as cyclohexyl, cycloheptyl or cyclooctyl groups, and aryl groups such as xylyl, tolyl and phenyl groups;
- a and b represent integers, a being 1, 2 or 3, b being 0, 1 or 2 and (a+b) being 2 or 3;

(ii) and optionally of at least one siloxy unit of formula (A2):

$$(Z)_cSiO_{(4-c/2} \qquad (A2)$$

in which:

- Z has the same meaning as above, and
- c represents an integer which is 2 or 3.

2) at least one organopolysiloxane B comprising, per molecule, at least two hydrogen atoms each bonded to a silicon atom, and preferably at least three hydrogen atoms each bonded to a silicon atom,
3) at least one hydrosilylation catalyst C,
4) at least one hydrosilylation reaction inhibitor D,
5) optionally at least one additive K, and
6) between 1.5% and 3.5% by weight, and preferably between 1.75% and 3.0% by weight, relative to the total weight of the silicone composition X, of at least one silicone resin Z having alkenyl groups bonded to silicon atoms and comprising:

a) at least one siloxy unit of formula (I):

$$Y\,R_aSiO_{\frac{(3-a)}{2}} \qquad (I)$$

in which:

- Y represents a $C_2$ to $C_6$ alkenyl group, and preferably a vinyl group,
- R is a monovalent hydrocarbon-based group chosen from alkyl groups having from 1 to 8 carbon atoms inclusive, such as methyl, ethyl, propyl and 3,3,3-trifluoropropyl groups, cycloalkyl groups, such as cyclohexyl, cycloheptyl or cyclooctyl groups, and aryl groups such as xylyl, tolyl and phenyl groups, and
- a = 0, 1 or 2, and preferably a = 1 or 2

b) at least one siloxy unit of formula (II):

$$R_bSiO_{\frac{(4-b)}{2}} \quad (II)$$

in which R has the same definition as above and b = 1, 2 or 3; and
c) at least one siloxy unit Q of formula (III):

$$SiO_{\frac{4}{2}} \quad (III)$$

with the following conditions:

a) the amounts by weight of the organopolysiloxanes A, B and Z are determined such that the value of the ratio $RH_{alk} = n_H/t_{Alk}$ is included in the following range: $0.10 \leq RH_{alk} \leq 0.80$, preferably in the following range $0.20 \leq RH_{alk} \leq 0.80$, and even more preferentially in the following range $0.20 \leq RH_{alk} \leq 0.75$, with $n_H$ = number of moles of hydrogen atom directly bonded to a silicon atom of the organopolysiloxane B and $t_{Alk}$ = number of moles of alkenyl directly bonded to a silicon atom of the organopolysiloxane A and of the silicone resin Z, and
b) the viscosities and the amounts by weight of the constituents of the silicone composition X are chosen such that the dynamic viscosity at 25°C of the silicone composition X is between 200 mPa.s and 100 000 mPa.s, and preferably between 200 mPa.s and 80 000 mPa.s.

**3.** Item that adheres to the skin according to Claim 1, **characterized in that** the silicone resin Z having alkenyl groups bonded to silicon atoms is chosen from the group consisting of:

- a silicone resin $Z^1$ of formula $MD^{Vi}Q$ in which:

• M is a siloxy unit of formula $R_3SiO_{1/2}$ in which R is a $C_1$ to $C_8$ alkyl or an aryl group such as xylyl, tolyl or phenyl,
• $D^{Vi}$ is a siloxy unit of formula $RR^1SiO_{2/2}$ with R being a $C_1$ to $C_8$ alkyl group or an aryl group and $R^1$ being a vinyl group, and
• Q is a siloxy unit of formula $SiO_{4/2}$;

- a silicone resin $Z^2$ of formula $MM^{Vi}Q$ in which:

• M is a siloxy unit of formula $R_3SiO_{1/2}$ in which R is a $C_1$ to $C_8$ alkyl or an aryl group such as xylyl, tolyl or phenyl,
• $M^{Vi}$ is a siloxy unit of formula $R_2(Vi)\ SiO_{1/2}$ with Vi being a vinyl group and R being a $C_1$ to $C_8$ alkyl group or an aryl group such as xylyl, tolyl or phenyl, and
• Q is a siloxy unit of formula $SiO_{4/2}$, and

- mixtures of the silicone resins $Z^1$ and $Z^2$.

**4.** Item that adheres to the skin according to Claim 1 or 2, **characterized in that** the silicone composition X comprises at least two different organopolysiloxanes B which are:

a) a chain extender $B^{ext}$ comprising:

• end monovalent siloxy units, which may be identical or different, of formula (M):

$$(H)_p(R^1)_qSiO_{1/2} \qquad (M)$$

in which the symbol $R^1$ corresponds to a $C_1$ to $C_8$ alkyl group; the symbol H represents a hydrogen atom and with p = 0 or 1, q = 2 or 3 and (p+q) = 3;

• divalent siloxy units, which may be identical or different, of formula (D):

$$(H)_n(R^2)_mSiO_{2/2} \qquad (D)$$

in which the radical $R^2$ corresponds to a $C_1$ to $C_8$ alkyl group or an aryl group, the symbol H represents a hydrogen atom and with n = 0 or 1, m = 1 or 2 and (n+m) = 2, and

• with the condition according to which the organopolysiloxane $B^{ext}$ comprises two hydrogen atoms each bonded to a different silicon atom per polymer, that is to say two Si-H functions per polymer;

b) a crosslinking agent $B^{ret}$ comprising:

• at least three siloxy units of formula (B.1):

$$(H)\ (L)_e\ SiO_{(3-e)/2} \qquad (B.1)$$

in which the symbol H represents a hydrogen atom, the symbol L represents an alkyl having from 1 to 8 carbon atoms inclusive or an aryl such as xylyl, tolyl or phenyl, and the symbol e is equal to 0, 1 or 2; and

• optionally other siloxy units of formula (B-2):

(L)gSiO(4-g)/2

in which the symbol L represents an alkyl having from 1 to 8 carbon atoms inclusive and the symbol g is equal to 0, 1, 2 or 3.

**5.** Item that adheres to the skin according to Claim 1 or 2, wherein the silicone composition X comprises at least two organopolysiloxanes A1 and A2 comprising, per molecule, at least two $C_2$ to $C_6$ alkenyl radicals each bonded to a silicon atom, **characterized in that**:

a) the organopolysiloxane A1 has a dynamic viscosity at 25°C of between 100 mPa.s and 120 000 mPa.s, and
b) the organopolysiloxane A2 is a gum having a consistency at 25°C of between 500 mm/10 and 1000 mm/10.

**6.** Item that adheres to the skin according to Claim 1 or 2, **characterized in that** the silicone composition X comprises at least three organopolysiloxanes A comprising, per molecule, at least two $C_2$ to $C_6$ alkenyl radicals each bonded to a silicon atom:

a) the first having a viscosity of between 100 mPa.s and 5000 mPa.s,
b) the second having a viscosity of between 5000 mPa.s and 15 000 mPa.s, and
c) the third having a viscosity of between 15 000 mPa.s and 100 000 mPa.s.

**7.** Item that adheres to the skin according to Claim 1 or 2, **characterized in that** the support S is composed of a nonwoven material, of a knitted or woven textile material, or of a plastic film.

**8.** Item that adheres to the skin according to Claim 7, **characterized in that** the support S is a perforated flexible polyurethane film or a continuous flexible polyurethane film.

**9.** Item that adheres to the skin according to Claim 1 or 8, **characterized in that** it comprises one or more layers N comprising an absorbent substance O, optionally separated by one or more intermediate layers P, placed on the support S on the side of the bottom face S2 of the support S.

**10.** Item that adheres to the skin according to any one of Claims 1, 2 and 8, **characterized in that** the support S is a

flexible polyurethane film and comprises, on at least one part of the bottom face S2, a pressure-sensitive adhesive.

**11.** Item that adheres to the skin according to any one of the preceding claims, **characterized in that** it is a medical device or an element of a medical device.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0633758 A
- EP 0633757 A
- EP 2001424 A
- EP 0300620 A
- FR 2971971 A1
- FR 3004990 A1
- WO 2011092404 A
- FR 1501350
- US 3508947 A
- EP 537086 A
- US 4933215 A
- US 4479987 A
- US 4974533 A
- US 3632374 A
- US 4830887 A
- EP 0737721 B1
- US 2676182 A
- US 3159601 A
- US 3159602 A
- US 3220972 A
- EP 0057459 A
- EP 0188978 A
- EP 0190530 A
- US 3419593 A
- US 3775452 A


### Littérature non-brevet citée dans la description


- FINAT Technical Handbook. 2001